# EUROPEAN PATENT SPECIFICATION

(11) **EP 2 353 628 B1**
(45) Date of publication and mention of the grant of the patent: **17.02.2021**
(21) Application number: 11151446.9
(22) Date of filing: 19.01.2011
(51) Int. Cl.: A61M 5/158, A61M 5/142, A61M 5/168, A61M 5/36, A61M 39/10

(54) **Modular infusion set with an integrated electrically powered functional component**
Modulares Infusionsset mit einer integrierten, elektrischen funktionellen Komponente
Ensemble de perfusion modulair avec élément fonctionnel électrique

(30) Priority: 28.01.2010 EP 10152026
(43) Date of publication of application: 10.08.2011
(73) Proprietor: F. Hoffmann-La Roche AG, 4070 Basel (CH); Roche Diabetes Care GmbH, 68305 Mannheim (DE)
(72) Inventor: Dogwiler, Jörg, 8962, Bergdietikon (CH); Geipel, Andreas, 4665, Oftringen (CH); Teutsch, David, 3270 Aarberg (CH); Huwiler, Christoph, 6415 Arth (CH)
(74) Representative: Rentsch Partner AG

(56) References cited:
- EP-A1- 1 818 664
- WO-A1-2009/116045
- WO-A1-2011/120661
- WO-A2-2008/114218
- WO-A2-2008/114220
- US-A1- 2009 234 272

## Description

The invention concerns an infusion set for attachment at an infusion site on a person's skin to administer a medicament delivered by a remote infusion pump. The invention is directed also to an infusion system comprising the infusion pump and the infusion set and furthermore to an infusion and monitoring system comprising the infusion system and a monitoring system for monitoring a parameter characteristic for the health condition of the person and relevant for the administration of the medicament. The invention is concerned in particular with diabetic therapy, i.e. the administration of insulin, but is advantageous also for other therapies and the administration of other infusible medicaments.

Infusion systems that deliver medication by infusion are typically divided into an infusion pump which can be carried on or under the clothing and an infusion set which can be attached at an infusion site directly on a person's skin such that a stiff or soft cannula projecting from an adhesive underside of the infusion set is placed with its tip in body tissue. Known infusion sets can conveniently be worn directly on the skin while the infusion pump including a medicament reservoir, feeding means, an energy source, control unit, manipulator, alarm means and tl1e like is worn remote from the infusion set. Such an infusion system is known, for example, from US 2009/0163355 A1. The small and lightweight infusion sets are replaced in short intervals for reasons of sterility, in diabetic therapy typically every two to three days, and must be inexpensive therefore.

WO 2008/114218 relates to subcutaneous infusion of drugs and use of energy sources to improve effectiveness of infused drugs. Excitation sources such as heat, cooling, vibrations, etc. are controlled and monitored in order to achieve a desired response. A treatment device can include a disposable part that includes the catheter and a reusable part which may include a processing unit and a power source. Sensors may be in the disposable part or in the reusable part that allow to measure required fluid parameters. WO 2008/114218 A2 discloses an infusion set comprising the combination of features of the preamble of claim 1.

WO 2008/114220 relates to subcutaneous infusion of drugs and use of energy sources to improve effectiveness. Using heat, cooling, vibrations, etc. a desired response is achieved. A drug delivery device includes a reusable part, such as a drug delivery pump, and a disposable part, such as an infusion set.

US 2009/0234272 discloses a device for stimulating tissue to improve the resorption kinetics of an active substance.

EP 1 818 664 relates to an insulin pump. A disposable adapter with a catheter provide the medicament fluid to the patient. The adapter is attached to the insulin pump. The adapter comprises a plastic membrane contacting the fluid and bending in case of an occlusion. The insulin pump comprises a light source and a sensor configured to detect the bending of the membrane.

WO 2009/116045 discloses a partially reusable dosing pump that can be worn on the body for subcutaneously delivering liquid. The dosing pump comprises a pump unit, a control unit, and a reservoir. The pump comprises a disposable section which snaps directly onto an infusion patch and a reusable section.

The functionality of infusion systems, not the least those for self-administration of the respective medicament, are ever increasing, and so do the safety requirements, despite the demand for keeping the associated costs low. Infusion systems may be coupled with monitoring systems for monitoring a biological parameter on which the dosed administration depends. US 2009/0163855 A1 discioses the combination of an infusion system with a separate glucose monitoring system which monitors a glucose ievei in subcutaneous tissue. The monitoring system comprises a monitoring set attached on the person's skin at a monitoring site locally independent from the infusion set.

US 2008/0200897 A1 and also US 2009/0062767 A1 arrange the infusion set and the monitoring set in a single infusion and monitoring set. The respective combined set comprises an infusion cannula and in addition thereto a further skin piercing element for sensing the glucose level in body tissue.

WO 2011/120661 A1 represents prior art under Article 54(3) EPC. It does not disclose a single lumen infusion cannula.

Measuring the glucose level in the direct neighbourhood of the infusion site bears the risk of adverse interferences. Administration might effect the measurement and the measurement, vice versa, might negatively effect the reception of the medicament. Furthermore, one of the advantages of the single-purpose infusion sets, the small size in terms of skin contact surface, is given up since the infusion cannula needs to be spaced from the sensor piercing element to reduce the cross-interferences.

It is an object of the invention to improve the administration of medicaments with infusion systems comprising an infusion pump and an infusion set which can be worn locally independent from the infusion pump at an infusion site directly on a person's skin, the infusion set being nevertheless small and inexpensive.

The invention is defined in claim 1. It is directed to an infusion set for administering a medicament delivered by an infusion pump which can be carried separately from the infusion set. In operation, when administering the medicament, the infusion set is attached directly at an infusion site on a person's skin while the infusion pump can be carried locally independent from the attached infusion set above or under the clothing, for example at a belt or in a pocket, fluidically connected with the infusion set by flexible tubing. Infusion pumps of this type are known e.g. from EP 0 991 440 B1, EP 0 985 419 B1, EP 1 633 414 B1 and EP 1 716 879 B1.

The infusion set comprises an adhesive underside for attachment at the infusion site on the skin, an infusion cannula projecting from the underside, and a first connector for fluidically connecting the cannula to the infusion pump to infuse the medicament delivered by the infusion pump. The infusion set can be fixed at the infusion site with its underside in adhesive contact with the skin surface. The infusion set can comprise a flexible pad which forms the adhesive underside. The adhesive underside can alternatively be formed e.g. directly on the underside of a relatively stiff base member of the infusion set. The cannula can either be a piercing needle or a soft cannula which is placed in the body tissue with the aid of a needle which is withdrawn after placement of the cannula. The cannula is arranged such that it is placed in the body tissue automatically together with placement of the infusion set on the skin. The cannula is preferably a subcutaneous cannula, i.e. it projects over the underside of the infusion set with a length for skin penetration and subcutaneous placement of a cannula tip. In such embodiments in which the infusion set is a subcutaneous infusion set, the cannula projects from the underside with a length of some millimetres, typically 4-12 mm.

The infusion cannula is the only skin piercing or penetrating element of the infusion set, and is a single-lumen cannula. A single-lumen cannula is advantageous as it facilitates the use of a cannula which is small in outer diameter. This helps to reduce perception of pain associated with penetration of the skin. If the cannula is placed in the body tissue with the aid of a needle, the needle is regarded as a part of the cannula, only aiding in placing the cannula in the body tissue. In operation of the infusion set, after having pierced or penetrated the skin, only the cannula is placed in the body tissue and the needle retracted or disposed. The infusion set is an infusion-only set which only serves the purpose to deliver the medicament. As an infusion-only set it is not equipped with means for sensing in the body tissue, in-vivo, any therapy relevant parameter characteristic for the person's health condition. A health characteristic parameter which can be used to control the feeding activity of the distant infusion pump, for example the glucose level in the blood or in subcutaneous tissue, can advantageously be measured by means of a separate monitoring system. Separating such a monitoring function from the infusion set facilitates to keep its size small, in particular its skin contact surface, and its weight and price low.

The infusion set nevertheless comprises at least one electrically powered functional component which improves medicament administration as such, for example by stimulating the body tissue at the infusion site or supervising the flow of the medicament through the infusion set. To relieve the infusion pump from providing the electrical energy necessary to operate the at least one functional component the infusion set includes its own energy source for electrically powering the functional component. The energy source can be provided e. g. as an electric battery or accumulator, a fuel cell, a photovoltaic cell, an electromagnetic generator for transforming motion energy into electrical energy or a thermo cell that transforms thermal energy from the patient's skin into electrical energy. A depleting energy source, like a battery, accumulator or fuel cell, may be supplemented by a non-depleting, regenerative energy source, like a photovoltaic cell, an electromagnetic generator or a thermo cell. In embodiments in which the infusion set comprises both types of energy sources the power management can be designed such that the functional component is powered by the depleting energy source during times where the regenerative energy source cannot generate electrical energy or only in insufficient amounts, and the regenerative energy source powers the functional component or supplements the depleting energy source during all other times. If the infusion set is equipped with a further or several further functional component(s) requiring electric power each further component is also electrically powered by this/those on-board energy source(s). An external energy source for electrical energy is not required to operate the single or plural electrically powered functional component(s). The infusion set is autonomous with respect to electric energy required for its own integrated operational functionality.

The infusion set is subdivided into a disposable part and a reusable part. The disposable part comprises at least one component or structure of the infusion set which requires short term replacement, and the reusable part comprises at least one component of longer operational lifetime. The disposable part can comprise structures or components which come into contact with body tissue. It forms the underside for attachment on the skin and comprises the infusion cannula and also the first connector for the fluidic connection with the infusion pump. The reusable part comprises at least the energy source and preferably a housing, e. g. a cap, for protectively accommodating the energy source. The disposable part further comprises a second connector and the reusable part a third connector, the second and third connectors mating one with the other to directly interconnect the disposable part and the reusable part such that both parts form a compact unit in the interconnected state.

The reusable part and the energy source or sources may constitute a unit which is replaceable only by a new reusable unit. The reusable part may alternatively accommodate the energy source such that the energy source, once depleted, can be replaced by a new one and the remainder of the reusable part be used together with the new energy source.

The infusion set of the invention improves therapy, the administration of the medicament, by means of the at least one electrically powered functional component. The improvement may reside, in particular, in affecting the body tissue at the infusion site, preferably in stimulating the body tissue, or in affecting or being affected by the medicament flow, preferably in supervising the medicament flow through the infusion set, or in providing an alert to indicate that replacement of the disposable or reusable part, of the functional component or the energy source is needed or some malfunction has been detected. Size and weight are nevertheless kept low due to the restriction of the infusion set to medicament delivery, to infusion only. The invention uses the word "or" in its usual logical meaning, i.e. as an "inclusive or", covering the meaning of "either ..... or" as well as the meaning of "and", as long as the respective context does under no circumstances allow for one of the two meanings only. So the infusion set may for example comprise, as a functional component, only a stimulator or only a delivery supervisory installation or only a lifetimer or may preferably comprise two or more different functional components, for example a lifetimer and either only a stimulator or only a delivery supervisory installation or all three of these example functional components, A preferred combination of functional components includes a delivery supervisory installation and also a stimulator. In even more advanced embodiments an alarm means, e.g. a buzzer, or a manipulator for manual manipulation of the infusion set is/are also disposed at the infusion set.

A preferred stimulator is a heating or vibratory stimulator for mechanically stimulating the tissue at the infusion site. The stimulator is preferably designed such that it affects the outer skin surface thereby stimulating the subcutaneous tissue. If the stimulator is a heating stimulator it can in particular be an ohmic resistor. The stimulator is preferably part of the underside of the disposable part. It can but must not be covered with adhesive material. A heating stimulator advantageously surrounds the infusion cannula at least partially, preferably all around. Its length and width, seen in a view onto the underside, is advantageously considerably larger than its thickness. If the stimulator is a mechanically stimulator, advantageously a vibratory stimulator, the same applies, e.g. such a stimulator is preferably located at the underside and may in particular surround the cannula at least partially. The stimulator can also be a heating and vibratory stimulator in combination, e.g. an ohmic resistor which is set in vibratory motion while heating at the same time or heating and vibrating, one operation timely separated from the other.

The at least one functional component can either be disposed at the disposable part or at the reusable part or can comprise a component part disposed at the disposable part and a further component part disposed at the reusable part. The expression "disposed at" comprises arrangements on an outer surface of the respective carrier part, namely the disposable or reusable part, as well as arrangements in which the respective component or component part is disposed in a recess or cavity of or is completely enclosed e. g. embedded in the respective carrier part. It defines, however, that the respective component or component part is a member only of that one of the two carrier parts at which it is disposed, once the two carrier parts have been disconnected one from the other. This explanation of "disposed at" holds not only with respect to the at least one functional component powered by the energy source but also with respect to any other kind of means, for example the energy source, disposed at either the disposable or the reusable part. Should the at least one functional component be disposed at the disposable part, the disposable and the reusable part, and thereby the electrically powered functional component and the energy source, can electrically be connected by means of a galvanic contact or by induction wherein the electric connection is preferably a direct connection between the disposable and the reusable part. A galvanic contact can for example be formed as a male and female connection, a plug and socket connection. A galvanic connection can alternatively be formed simply as a pressure contact, the pressure being exerted by an elastic resilience of either only a connecting means of the disposable part or the reusable part or an elastic resilience of the connecting means of both parts, wherein the pressure exerted in the contacted state is advantageously parallel to a direction into which the reusable part is attached to the disposable part to establish the interconnected state of these parts. The electric connection is established in all embodiments requiring for the transmission of electrical power preferably automatically by interconnecting the disposable and the reusable part. An additional manual action is advantageously not required. The connecting means for the mechanical interconnection and the connecting means for the electric connection are in all embodiments designed and arranged preferably such that both kinds of connections are established by the same relative movement the two module parts have to accomplish for being interconnected.

The infusion set can comprise, as already mentioned, a delivery supervisory installation as the only or one of a plurality of functional component(s). The delivery supervisory installation supervises the delivery of the medicament by sensing a characteristic variable of the medicament delivery or more specifically of the medicament flow, for example the rate of flow, possible air bubbles in the flow or the fluid pressure. The delivery characteristic variable, preferably flow characteristic variable, can be sensed within or at the infusion cannula. The variable can instead or in addition be sensed upstream of the cannula and downstream of or directly at the first connector, the fluidic connector of the infusion set. The delivery characteristic variable can in particular be sensed with the aid of a sensor which is in contact with or disposed in a medicament feeding line extending from an upstream end of the first connector to the cannula tip. Such a sensor is referred to in the following as a contact sensor element or unit. The contact sensor element or unit is advantageously in direct contact with the feeding line or the medicament. It can be disposed on an outer circumferential surface of the feeding line. It can alternatively be embedded in the feeding line or attached to the inner surface of the feeding line or, as a further alternative, be an integrated part of the feeding line.

A delivery supervisory installation supervises in first embodiments the flow of the medicament between the upstream end of the first connector and the upstream end of the cannula. In second embodiments a delivery supervisory installation is located between the upstream end of the cannula and the cannula tip.

Supervision of a delivery characteristic variable can advantageously be accomplished by means of a contact sensor element or unit which experiences a deformation, preferably an elastic deformation, in dependence on the fluid pressure in the feeding line. The deformation or degree of deformation is detected by a further sensor means, preferably without direct contact, i. e. by a non-contact sensor. The detection can preferably be an optical one. The contact sensor element or unit is preferably disposed at the disposable part and the non-contact sensor preferably at the reusable part. Detection is accordingly performed via outer surfaces of the two module parts, these surfaces facing each other and being internal surfaces of the infusion set in the interconnected state of the disposable and the reusable part. A sensor means of the disposable part, for example the mentioned contact sensor element or unit, can advantageously be passive, i.e. does not need to be powered. A suitable alternative contact sensor element or unit would be e.g. a strain gauge which, if disposed at the disposable part as preferred, would require a power transmission via an electric interface between the disposable and the reusable part.

The contact sensor element or unit can advantageously comprise a micro-fluidic chamber having a bottom substrate and a top cover, the top cover being spaced from the bottom substrate so as to define a height (H1) of the chamber. One or more walls or fillings can be positioned in the chamber, the walls or fillings defining a fluid channel there between such that the fluid channel extends from an inlet of the chamber to an outlet of the chamber. Each of the walls or fillings has a height (H2) less than the height (H1) of the chamber so as to define a fluid gap between a top surface of each wall or filling and the top cover. The dimensions (HI, H2) of the walls or fillings and the chamber are chosen such that the fluid gap will be filled with liquid by capillary forces via the fluid channel when liquid is introduced into the fluid chamber. The fluid gap adjacent to a section of the fluid channel filled by a liquid introduced into the fluid chamber will be filled with said liquid by capillary force. The top cover is preferably a flexible, resilient membrane deforming in dependence on the medicament fluid pressure.

An advantageous delivery supervisory installation is a pressure sensor comprising the micro-fluidic chamber and a detecting means that is arranged to measure the deformation of the top cover of the micro-fluidic chamber. At least a part of the surface of the bottom structure or the walls or the top cover facing toward an inner volume of the chamber can be hydrophilic. The fluid channel can advantageously have a meander-like shape. The non-contact sensor mentioned above is preferably arranged to measure a deformation of the top cover of the micro-fluidic chamber. Alternatively or preferably in addition such a micro-fluidic chamber can aid in degassing the medicament fluid thereby serving as a degasser. The top cover can be gas-permeable for degassing. In a modification the chamber is of dimensions above the micro range, so capillary forces do not play a role or decisive role. A meander-like shape of the feeding line is nevertheless advantageous for increasing the flow resistance in the region of the sensor membrane.

Another type of a contact sensor element or unit which is preferably operated as a pressure sensor comprises a stack of layers, preferably coplanar layers, with (a) a rigid top layer and a rigid base layer, (b) a resilient metallic electrode layer and a metallic counter-electrode layer, the electrode layer and the counter-electrode layer being electrodes of a sensing capacitor, and (c) a spacer layer which has a through cut-out, the through cut-out defining an electrode cavity, wherein the fluid channel is coupled to the electrode layer such that a fluidic positive pressure of the drug in the fluid channel causes the electrode layer to bend into the electrode cavity, thus modifying the capacitance of the sensing capacitor. The contact sensor element or unit of this type constitutes a delivery supervising sensor on its own. It is expediently disposed at the disposable part and connected electrically with the energy source via an electrical connection of the two module parts.

At least two electrodes can be provided. At least one of the electrodes can be a subcutaneous electrode comprised by the infusion cannula. The other of the at least two electrodes can also be disposed at the cannula, in particular also as a subcutaneous electrode. This at least one other electrode can instead be disposed in the feeding line upstream of the cannula either still at the infusion set or at the infusion pump or in the tubing for fluidically connecting the infusion set with the infusion pump. An electrode disposed in the tubing or at the pump can be operated with successive subcutaneous electrodes which get disposed with the disposable part of the infusion set. An impedance measuring means is operatively coupled to the at least two electrodes and is further designed to measure at least one impedance value between the at least two electrodes. An event trigger means is operatively coupled to the impedance measuring means and is designed to evaluate the at least one impedance value and to generate an event trigger if evaluation of the at least one impedance value indicates the occurrence of a delivery anomaly. At least one of the impedance measuring means and the event trigger means can be disposed at the reusable part of the infusion set.

The term "impedance" is used in the sense of an electrical impedance which may comprise ohmic, as well as capacitive or inductive components. Accordingly, the term "impedance value" refers to a complex value or a vector of values reflecting either or all of the impedance components. In some preferred embodiments, the impedance is an ohmic resistance and the corresponding impedance value is a resistor value. However, capacitive or inductive impedance components may be evaluated alternatively or additionally to an ohmic impedance component. The term "impedance value" may further be referred to as a value correlated with and derivable from an impedance or impedance component, such as a specific conductivity, capacity, or the like as well as to an electrical measuring value correlated with an impedance or impedance component, such as the voltage drop over an impedance. The impedance is especially defined by the subcutaneous tissue or the drug which is administered by the infusion device. The term "subcutaneous electrode" refers to an electrode which is placed in the subcutaneous tissue. Such a subcutaneous electrode may be arranged inside the infusion cannula or at the outer surface of the infusion cannula in an area which is placed in the subcutaneous tissue during application.

In preferred variants, the at least one subcutaneous electrode and preferably the at least one further electrode is/are, completely or partly, coated by substantially inert layers of gold, silver, platinum, or the like or may be made from substantially inherent materials.

The contact sensor for impedance measurement is best understood based on a consideration of the subcutaneous tissue impedance, in particular the specific ohmic resistance of the subcutaneous tissue and of the medicament fluid. The specific ohmic resistance of the subcutaneous tissue and thus the ohmic resistance which may be measured between two electrodes placed in the subcutaneous tissue is under normal conditions largely given by the specific ohmic resistance of the interstitial fluid. The specific ohmic conductivity, i.e., the reciprocal of the specific ohmic resistance, of the interstitial fluid is about 15.8 mS/cm. The specific ohmic conductivity of insulin formulations and a number of further liquid medicaments is about 2 mS/cm, that is, several times smaller as compared to interstitial fluid. In a stationary state with no medicament being administered or having been administered for some time, the specific impedance and in particular the specific ohmic resistance of the subcutaneous tissue is largely determined by interstitial fluid. If medicament is administered, the interstitial fluid is, at least partly, temporarily displaced by medicament fluid in an area around the administration aperture at the distal tip of the infusion cannula, the medicament forming a subcutaneous medicament depot. Accordingly, the specific impedance and in particular the specific ohmic resistance of the subcutaneous tissue around the infusion cannula shows a temporary variation, the temporary variation especially involving a temporary increase and a peak in the specific ohmic resistance. After completing the administration, the specific impedance returns to its initial value along with the medicament being absorbed by the subcutaneous tissue. Variation of the specific impedance with respect to time is reflected by a corresponding variation of a impedance value measured between two subcutaneous electrodes which are located at fixed positions in the subcutaneous tissue.

In addition, the medicament concentration during and immediately after administration decreases with increasing distance from the administration aperture. During and immediately after administration, the specific impedance of the subcutaneous tissue is therefore non-uniform with respect to position. The specific ohmic resistance decreases with increasing distance from the administration aperture. Accordingly, the specific ohmic resistance or any component of the tissue impedance may therefore be considered as scalar field having a specific impedance gradient. In the case of no impedance gradient, that is, in case of a uniform spatial impedance distribution, the impedance value measured between two subcutaneous electrodes is substantially proportional to the distance between the electrodes. This is not the case if an impedance gradient is present, that is, for a non-uniform spatial impedance distribution. The specific impedance variation along a given axis may therefore be determined by measuring impedance values between at least three electrodes of given distances placed along the given axis. Variation of the specific impedance along the axis is reflected by the impedance values measured between pairs of the at least three electrodes not being proportional to the distance between the electrodes. The axis may advantageously be the cannula axis. For simplicity reasons, the term "impedance distribution" is used here for the spatial distribution of the specific impedance of the subcutaneous tissue.

The variability of the specific impedance with respect to time or position may be evaluated for administration supervision and administration anomaly detection. If, for any reason such as an occlusion, a leakage, a disconnected infusion cannula, a device fault, or the like, the medicament is not administered, this variability will not occur. In preferred embodiments, the impedance measuring means therefore is designed to monitor the at least one impedance value as a function of time or position, and the event trigger means is designed to generate an event trigger if the at least one impedance value as a function of time or position indicates the occurrence of an administration anomaly.

The infusion set can be equipped with a lifetimer, either alternatively or preferably in addition to one or more of the other functional components mentioned here. The lifetimer can be designed such that it alerts the person at expiration of a predetermined time of use of the disposable part e.g. to remind the person that the disposable part needs regular replacement. Alternatively or more preferred in addition the lifetimer may be designed to alert the person that the emergy source is depleted already or will reach a predetermined end of operational lifetime requiring for replacement only of the energy source or of the disposable part as a whole. The alert is preferably an acoustic or vibratory alert or both either in combination or sequentially one after the other, for example in alternation. If a vibratory stimulator is present, the alert should be distinct from the vibratory stimulating operation of such a stimulator. The lifetimer includes a clock which is started either manually by actuating an optional actuator at the time the disposable part is attached on the skin or automatically by establishing the interconnection of the disposable part and the reusable part or by establishing the interconnection of the disposable part and the tubing.

The infusion set may comprise a manipulator to actuate the at least one or at least one of a plurality of on-board functional component(s). The manipulator can be a key or button, for example a turn-button or in particular be a push-button, or a squeeze element or some other type of actuator for manual activation. A manipulator can be disposed at the infusion set alternatively or in addition to a remote manipulator e. g. of the infusion pump. The manipulator is disposed preferably at the reusable part.

A further alternative or additional functional component is a motion sensor detecting and preferably also recording motional activity of the user. A motion sensor can for example be formed by an acceleration sensor. The motion sensor may serve the purpose to assess the patient's physical activity e.g. for record keeping purposes or for controlling the administration. For example, a physical activity may be detected and, depending thereon, a temporary reduction of a basal administration be recommended to the patient or automatically carried out. With a motion sensor it can for example be detected if the patient is awake or asleep. The detection of exceptional events e.g. a mechanical shock might be entered to the device history. The lifetimer or the motion sensor, if present, is or are preferably disposed at the reusable part.

A further alternative or additional functional component is a temperature sensor e.g. to sense ambient temperature and thereby detect possible exposition to exceptionally high or low temperature that can be entered into a storage means for the device history. A temperature sensor can additionally or alternatively be disposed such that correct attachment of the infusion set to the skin can be supervised by means of such a temperature sensor which can advantageously be disposed at the underside of the disposable part to contact the skin or to be in close vicinity of the skin. A temperature sensor can alternatively serve the purpose to supervise a stimulator for stimulating the body tissue by heating.

A further alternative or additional functional component is a skin contact sensor which, besides the temperature sensor as mentioned above, may be a skin impedance sensor or an electrical switch contact that is closed or opened by the skin contact.

A humidity or liquid sensor for detecting medicine leakage or for supervising correct operation of electrical contacts, if the disposable part comprises electrically powered components, is also an option as an alternative or additional functional component.

Another alternative or additional candidate as a functional component is a perspiration sensor sensing and preferably recording perspiration directly at the contact interface between the underside of the infusion set and the skin. A perspiration sensor can for example comprise electrodes located at the underside of the disposable part measuring the electrical conductivity on the surface of the skin.

The infusion set can advantageously comprise an alarm means, preferably for releasing an acoustic or vibratory alarm. The alarm means can be part of or coupled to a stimulator or a delivery supervisory installation or a lifetimer or some other kind of functional component. The alarm means can also be coupled to and shared by two or more different functional components of the infusion set. The alarm means, if present, is preferably disposed at the reusable part.

In preferred embodiments the infusion set comprises a signal processing or control unit which can be designed either only for on-board processing of signals from an on-board functional component formed as a sensor means or only for controlling the operation of an on-board functional component e. g. the stimulator or a sensor means. The signal processing or control unit is preferably a signal processing and control unit for both signal processing and controlling. The processing or control unit is preferably disposed at the reusable part. The signal processing function can e. g. reside in transforming analogue signals of a sensor means in digital data which can be transmitted to the pump. The signal processing or control unit can include a data storage means for storing digital data. It can include a processing unit e. g. for recognition of hazardous situations sensed with the aid of a delivery supervisory installation. Such a processing capability would relieve the infusion pump if alarms are to be created by the pump and would simplify integration of the infusion set in the control system of the pump.

If the infusion set comprises sensor means, e. g. a delivery supervisory installation, it can be advantageous to amplify the outgoing sensor signals on-board the infusion set. If such a sensor means is disposed at the disposable part a signal amplifier can be disposed at the disposable part to transmit amplified signals to the infusion pump or to the signal processing or control unit mentioned above.

The disposable part is in preferred embodiments designed to be capable to deliver the medicament at the infusion site without the reusable part being interconnected. Feeding of the medicament is in such preferred embodiments solely via the disposable part. Most preferred, the reusable part is fluidically isolated from the disposable part and has no physical contact with the medicament. The disposable part can in such embodiments be used without the reusable part. This applies irrespective of the kind(s) of functional component(s) the infusion set is equipped with for improving therapy, e.g. for enhancing the reliability that the medicament dose is correctly delivered or for improving reception of the medicament in the tissue or alerting the person that the disposable part has reached its end of operational life for which it was designed.

A disposable part designed to be capable to deliver the medicament at the infusion site in fluidically bypassing the reusable part or without the energy source of the reusable part or any electrical or optical coupling with the reusable part or even without the reusable part being interconnected is advantageous not only for isolating the reusable part fluidically from the disposable part but also e.g. for using the infusion set or only the disposable part in combination with an infusion pump which has not the capability to operate in adapted combination with the one or more functional components electrically powered by the energy source of the reusable part or does not support the one or more functional components or is even incompatible to said one or more functional components of the infusion set. For such infusion pumps the reusable part can even be put aside by the user or can be omitted, i.e. the infusion set be delivered with only the disposable part. Producing infusion sets with the disposable and the reusable part and also infusion sets with only the disposable part can furthermore reduce the production costs because of production scale effects, i.e. higher production numbers of the disposable parts. Under these circumstances two different infusion sets are available, namely a first infusion set comprising the disposable and the reusable part of the claimed invention and a further infusion set. This further infusion set can consist solely of the disposable part disclosed under the claimed invention or can comprise such a disposable part and a further part designed to be substitutional for the reusable part of the claimed invention. This further part may serve simply as a cover for the disposable part. In such embodiments this further part is expediently designed to be disposable together with the disposable part of the claimed invention. The further part can in particular comprise connecting means similar or identical to that of the reusable part of the invention for a mechanical interconnection with the disposable part of the claimed invention. This further part may serve as a covering part which covers in the interconnected state the upper side of the disposable part of the claimed invention. This covering part can advantageously provide for a smooth upper surface of the further infusion set.

The disposable part and the reusable part can be interconnected plainly mechanically, in particular if the disposable part does not comprise a functional component which needs electric energy. In preferred embodiments, however, the two parts are interconnected not only mechanically but also electrically, electrically in particular by direct galvanic contact or a direct contactless interface for transmittance of energy to operate an active functional component of the disposable part. An optical interconnection can comprise one or more optical fiber(s) interconnected physically, or it can be some other type of an optical interface allowing optical beams to pass through e.g. simply a window or breakthrough. The interconnection can alternatively be only a mechanical and optical one, for example as described above in connection with a preferred embodiment of a delivery supervisory installation. In further developed embodiments the disposable part is interconnected with the reusable part mechanically, electrically and optically.

The infusion set can advantageously comprise a transmitter for transmitting signals to the infusion pump, for example an alarm signal if an alarm means is not present at the infusion set or for creating an alarm in addition at the infusion pump. The transmitter may alternatively or in addition to transmitting an alarm signal be capable of transmitting sensor signals or data, for example signals or data of a delivery supervisory installation or some other type of sensor like the mentioned motion sensor or perspiration sensor.

The infusion set may comprise a receiver, either alternatively or preferably in addition to a transmitter, for receiving signals from the infusion pump or an optional extra monitoring system for monitoring a therapy relevant health condition of the person, for example by monitoring the glucose level. A receiver can for example be coupled with the stimulator, if present, such that the infusion pump or the extra monitoring system can automatically or the person can manually activate the stimulator at the infusion pump or with the aid of some other remote control unit. A delivery supervisory installation or some other sensor means on-board the infusion set might also or alternatively be designed for a remote activation via the on-board receiver.

The transmitter or receiver or an on-board transceiver is preferably designed for wireless communication as for example via IR or RF like Bluetooth. Signal transmission, if present, can alternatively or in addition be accomplished by means of the flexible tubing which connects the infusion set and the infusion pump fluidically in use of the infusion system. Wired signal or data transmission can in particular be performed optically e.g. via optical fibres on or preferably embedded in the flexible tubing. Such a solution requires, of course, optical coupling means of the infusion pump as well as of the infusion set.

The infusion set may comprise flexible tubing for connecting the infusion set fluidically with the infusion pump. The tubing can in particular be connected directly with the disposable part. The tubing can comprise an upstream connector for connecting the tubing releasably with the infusion pump and a downstream connector for connecting it releasably with the infusion set. In alternative embodiments also including flexible tubing for the interconnection with the infusion pump such tubing can be connected, unreleasably, with the infusion set and comprise a connector for a releasable connection with the infusion pump only at its upstream end. In other embodiments the infusion set can furthermore comprise an ampoule or some other type of reservoir prefilled with a medicament. In such embodiments the ampoule or other type of reservoir will or can be disposed together with the disposable part of the infusion set. The tubing can in such embodiments be connected unreleasably either with the infusion set or the ampoule or other type of reservoir, or both, or can alternatively be connected via respective connectors releasably with the infusion set and also releasably with the ampoule or other type of disposable reservoir.

An infusion set which is modular in that a disposable part is combined with a reusable part and comprises at least one of the functional components disclosed here, in particular the stimulator or the delivery supervisory installation, is advantageous also without the on-board energy source. Such a modified modular infusion set can advantageously be designed as disclosed under the invention claimed here except that it is electrically powered by an external energy source e. g. an energy source disposed at the infusion pump and electrically connected with the at least one electrically powered functional component via the fluidic tubing. The energy source can in principal be disposed at some other device external to the infusion set, for example at an external energy module for electrically powering only the infusion set, i.e. its at least one functional component, or for electrically powering the infusion set and the pump as well. The applicants preserve the right to claim a modular infusion set on its own and in combination with an external infusion pump regardless of the question where the energy source is disposed at.

Preferred features of the invention are also described in the sub-claims and combinations of these. The features described in these, and those explained above, reciprocally complement each other in an advantageous way.

The invention is explained below on the basis of example embodiments. Features explained by way of the example embodiments, each individually and in the disclosed combinations of features, advantageously develop the subjects of the claims and also the features explained above. There is shown:
- Fig. 1:: an infusion set of a first embodiment of the invention with a disposable and a reusable part in an interconnected state;
- Fig. 2:: the infusion set with the reusable part disconnected from the disposable part;
- Fig. 3:: components of the infusion set in a disassembled state;
- Fig. 4:: the infusion set in a section;
- Fig. 5:: a diagrammatical illustration of a first embodiment of an infusion system including the infusion set;
- Fig. 6:: a diagrammatical illustration of a second embodiment of an infusion system;
- Fig. 7:: a diagrammatical illustration of a third embodiment of an infusion system;
- Fig. 8:: a diagrammatical illustration of a fourth embodiment of an infusion system;
- Fig. 9:: a contact sensor element or unit of a delivery supervisory installation in a top view;
- Fig. 10:: the contact sensor element or unit in the cross-section A-A of figure 8;
- Fig. 11:: a detail view of figure 10;
- Fig. 12:: a non-contact sensor of the delivery supervisory installation;
- Fig. 13:: a modified non-contact sensor;
- Fig. 14:: a delivery supervisory installation in an alternative embodiment;
- Fig. 15:: a modification of the delivery supervisory installation of figure 14;
- Fig. 16:: a schematic structural view of a further embodiment of an infusion system;
- Fig. 17:: an infusion system of the invention; and
- Fig. 18:: a cross-section of a subcutaneous portion of an infusion cannula comprising a subcutaneous contact sensor element or unit.

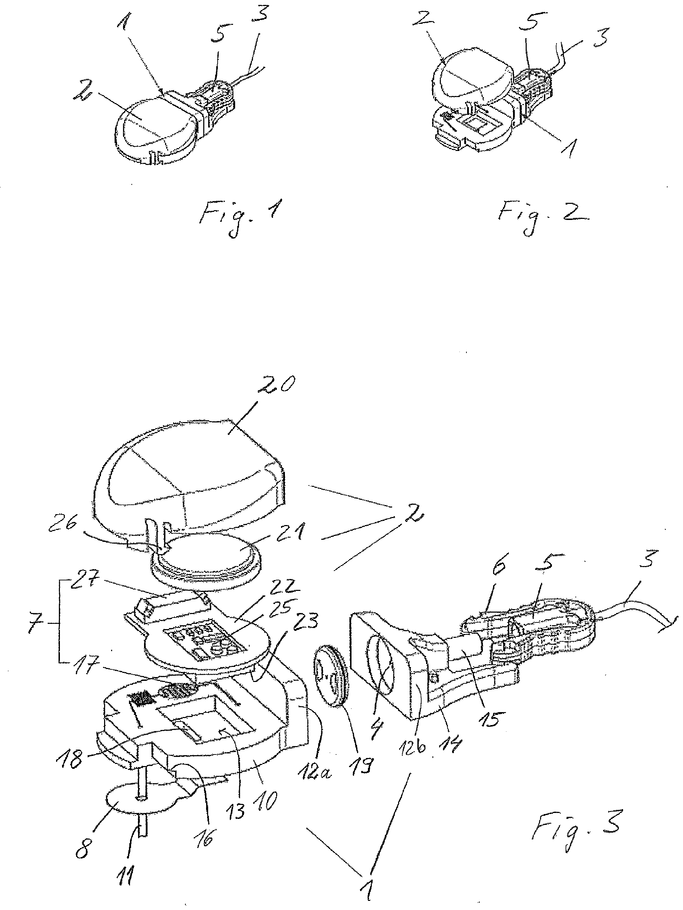

Figure 1 shows a first embodiment of an infusion set which can be attached on the skin of a person to administer a medicament fluid - in the following medicament - into the body tissue at the infusion site through an infusion cannula which projects from the underside of the infusion set. The underside of the infusion set is covered with an adhesive to fix the infusion set on the skin. The infusion set is modular in that it comprises a disposable part 1 forming the underside which is the skin contact surface of the infusion set, and a reusable part 2 which is releasably interconnected with the disposable part 1. The infusion set is shown in figure 1 with the two module parts 1 and 2 in the interconnected state. The interconnection is a direct interconnection, i.e. the two parts 1 and 2 are mechanically interconnected, one mechanically directly with the other. The infusion set 1, 2 is fluidically connectable to an infusion pump by means of flexible tubing 3. In operation, when medicament is infused, a feeding means of the infusion pump feeds the medicament from a reservoir of the infusion pump via the tubing 3 to the infusion set 1, 2 and via the cannula into the body tissue. The cannula can in particular be a subcutaneous cannula projecting from the underside of the infusion set with a length suitable for subcutaneous delivery of the medicament. The flexible tubing 3 is fluidically and mechanically connected to the infusion set 1, 2 by means of a fluidic connector 5. The connector 5 constitutes the downstream end of the tubing 3. A similar connector may constitute the upstream end of the tubing 3 to releasably connect the tubing 3 with the infusion pump which may be worn remote from the infusion set 1, 2. In modifications the downstream connector 5 of the tubing 3 may be omitted and the tubing 3 be unreleasably connected with the infusion set 1, 2. In such modifications the upstream connector of the tubing 3 can constitute the first connector of the infusion set 1, 2. In still further embodiments the tubing 3 can be connected unreleasably with the infusion set 1, 2 and furthermore be connected directly with an ampoule prefilled with the medicament or some other type of reservoir prefilled with the medicament such that the ampoule or other type of reservoir is disposed off, after use, together with the disposable part 2. In such embodiments the ampoule or other type of reservoir can constitute the first connector of the infusion set, however, more preferred, the tubing 3 is provided with a downstream connector, e.g. the connector 5 for a releasable connection with the infusion set 1, 2. In all embodiments in which the tubing 3 comprises a downstream connector for fluidically connecting the tubing 3 with the infusion set 1, 2 this connector and a counter connector of the infusion set 1, 2 may be designed such that a tubing 3 which is provided separately from the infusion set 1, 2 or as a separate part of the infusion set 1, 2 can be connected with the disposable part 2 only once. In such a modification the mechanical interconnection once established is unreleasable in order to prevent that the tubing 3 can be reused without the disposable part 2.

Figure 2 shows the infusion set 1, 2 with the reusable part 2 disconnected from the disposable part 1. The disposable part 1 is still connected with the tubing 3 by means of the connector 5. Medicament can still be infused while the reusable part 2 is disconnected since the medicament flow is through the tubing 3 and only the disposable part 1. The reusable part 2 is fluidically isolated from the flow of the medicament.

The two module parts 1 and 2, as can be seen in figure 1, are forming a low profile, i.e. a flat infusion set 1, 2 in their interconnected state. The reusable part 2 is located in the interconnected state on top of the disposable part 1 to reduce the area of the infusion set 1, 2 when seen in a top view onto the upper side of the infusion set 1, 2. The total area covered by the infusion set 1, 2, in the skin attached state is roughly the same as that of the disposable part 1 alone.

In figure 3 the infusion set 1, 2 is illustrated in a disassembled state of components of which it is composed of. The disposable part 1 constitutes a basic structure of the infusion set and comprises a primary base member 10 and a secondary base member 14 which are formed separately and jointly fixed one to the other to form the unitary base structure 10, 14. The base members 10 and 14 form a flat base comprising the adhesive underside for skin attachment and an upper side opposite to the underside. Base member 14 is formed with a first connector 15, a fluid connector, for releasably connecting the connector 5 to the disposable part 1 such that medicament can flow via the fluidic connection of the interconnected connectors 5 and 15 into a feeding line 4 in which the medicament is fed only via the disposable part 1 to an upstream end of the cannula 11. The feeding line 4 and the cannula 11 can advantageously be formed as a unitary part that is embedded in the base member 10 and leaves the base member 10 via a curvature at the underside to constitute the cannula 11. The upstream end of the cannula 11 is regarded to be located at the underside of the disposable part 1, flush with the underside. Regardless of how the feeding line 4 and the cannula 11 are formed, when speaking of the cannula 11 only the projecting length is meant.

Base member 10 comprises an upright structure 12a projecting from the upper side of base member 10, and base member 14 comprises an upright structure 12b projecting from the upper side of base member 14. In the assembled state the base members 10 and 14 are joint together with their upright structures 12a and 12b being fixed one to the other. The fluid connector 15 projects from the upright structure 12b in a small distance from the upper side of the base member 14 and parallel to the underside of the disposable part 1 such that connector 5 can be interconnected with connector 15 in a relative motion parallel to the underside of the disposable part 1. Connector 5 comprises locking means 6 which automatically interconnect mechanically with counter locking means of the disposable part 1, in the example embodiment with counter locking means of the base member 14. The base members 10 and 14 can in alternative embodiments be formed in a single piece, however, forming the basic structure 10, 14 in two or even more different pieces facilitates the forming process and offers the opportunity to dispose an air degasser 19 in the medicament flow. Air degasser 19 is accommodated in a chamber which is jointly formed by the base members 10 and 14.

The infusion set 1, 2 comprises functional components to improve the fluid flow function and reception of the medicament at the infusion site. One of the functional components is a delivery supervisory installation 7 for supervising the flow and hence the delivery of the medicament, and another one is a stimulator 8 for stimulating the body tissue at the infusion site to improve the reception of the medicament in the body tissue. The stimulator 8 is a heating means for stimulating the body tissue by heating the skin in direct heat contact. The delivery supervisory installation 7 is a flow detector for supervising the flow of the medicament through the feeding line 4.

The stimulator 8 is an electric (ohmic) resistor disposed at the underside of the disposable part 1 in direct vicinity of the cannula 11. The cannula 11 projects, as preferred, through a passage formed in the stimulator 8 which closely and completely surrounds the cannula 11 directly at the underside of the disposable part 1. The stimulator 8 is a flat plate-like structure or a foil structure.

The stimulator 8 is electrically powered by an energy source 21, an electrical battery or accumulator, which is an internal on-board energy source of the infusion set 1, 2. The energy source 21 is a flat and disc-like body. The energy source 21 is accommodated in a chamber of the reusable part 2, the chamber being formed by a cap 20 and a base member 22 of the reusable part 2. Cap 20 forms a top surface of the reusable part 2 and infusion set 1, 2 oppositely facing away from the underside of the disposable part 1. Base member 22 forms an underside of the reusable part 2, this underside being in direct contact with the upper side of a flat base portion of the basic structure 10, 14 of the disposable part 1 when the parts 1, 2 are in the interconnected state.

The reusable part 2 furthermore comprises an electronic control unit 25 controlling the stimulating action, heating action in the example embodiment, of the stimulator 8. The stimulator 8 is electrically connected to the control unit 25 via an electric connection means 18 of the disposable part 1 and is electrically connected to the energy source 21 via the control unit 25. Control unit 25 is disposed at the reusable part 2, in the example embodiment accommodated in the reusable part 2 and disposed at the base member 22.
The disposable part 1 and the reusable part 2 are mechanically directly connected one to the other by means of a releasable interlocking action of mechanical connectors 16 of the disposable part 1 and mechanical counter connectors 26 of the reusable part 2. The connectors 26 are formed as hooks which grip behind the connectors 16 when the reusable part 2 is brought at its underside in contact with the upperside of the flat base portion of the disposable part 1. The energy source 21 is galvanically connected with the stimulator 8 automatically at the same time the mechanical interconnection of the parts 1 and 2 is established.

The delivery supervisory installation 7 comprises a contact sensor element or unit 17 disposed at the disposable part 1 and a non-contact sensor 27 disposed at the reusable part 2. The non-contact sensor 27 is accommodated in the chamber formed by the base member 22 and cap 20 of the reusable part 2. Contact sensor element or unit 17 is disposed to be in optical contact with the non-contact sensor 27 when the two module parts 1 and 2 are interconnected. Contact sensor element or unit 17 comprises or consists of an elastic membrane which is deformed in dependence on the fluid pressure in the feeding line 4. The non-contact sensor 27 detects the state of deformation of the contact sensor element or unit 17 and thus flow of the medicament through the feeding line 4. The contact sensor element or unit 17 is passive, i.e. it does not require power supply. Non-contact sensor 27 is the active part of delivery supervisory installation 7 and is electrically powered by the energy source 21, i.e. completely internal of the reusable part 2. It is powered via the control unit 25.

The control unit 25 controls the operation of delivery supervisory installation 7 and also the operation of the stimulator 8. It is furthermore a signal processing means as it processes the output signals received from the non-contact sensor 27. This processing can in particular be or comprise transforming analogue output signals of the non-contact sensor 27 into digital data. The processing capability can preferably include outputting data to the infusion pump. In principal, the signal processing function of the control unit 25 can be restricted to outputting analogue sensor signals to the infusion pump, however transforming these signals into digital data on-board the infusion set and outputting these data or only part of these data is more preferred. The control unit 25 can include a data storage means for storing of the sensor data or only of selected data, i.e. event data, after further processing. Event data can for example be data representing the exceedance of a predetermined threshold level of e.g. the fluid pressure.

The electrical energy required for these operations and also the operation of the control unit 25 is provided alone by the internal energy source 21. The infusion set 1, 2 is electrically self sustaining, it does not need electrical energy from outside, for example the infusion pump. Expensive parts of the modular infusion set 1, 2, namely the energy source 21 and in particular the control unit 25 and the non-contact sensor 27, are integral to the reusable part 2 whereas fluid flow communication between the downstream end of tubing 3 and the cannula 11 is assigned completely to the disposable part 1. The disposable part 1 can be used without the reusable part 2 comparable to infusion-only conventional infusion sets.

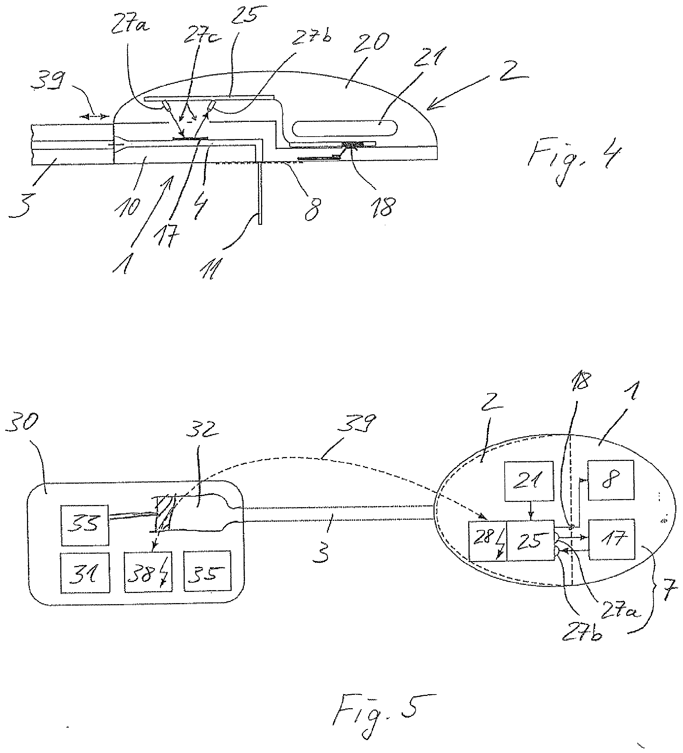

Figure 4 shows the infusion set 1, 2 in a section along the feeding line 4. The stimulator 8 is accommodated in a flat recess at the underside of the disposable part 1 to form this underside as a continuously smooth contact surface, The electrical connecting means 18 is resiliently urged against a contact means of the reusable part 2 to galvanically connect the stimulator 8 via the control unit 25 to the energy source 21. The contact sensor element or unit 17 is attached to the outer circumferential surface of the feeding line 4. It may alternatively form part of the feeding line 4. It is elastically deformed in dependence on the fluid pressure within the feeding line 4. The non-contact sensor 27 is of the optical type. It comprises an optical emitter 27a, for example a LED, which directs an optical beam onto the contact sensor element or unit 17, and an optical receiver 27b which receives the reflected beam as an optical signal. The optical beams are transmitted via the contact surfaces of the disposable and reusable part 1 and 2, i.e. via the underside of the reusable part 2 and the upper side of the disposable part 1 which together form this optical interface 27c by direct contact. The optical elements, emitter 27a and receiver 27b, are electrically powered by the energy source 21 and operationally controlled by the control unit 25.

The disposable part 1 and the reusable part 2 are interconnected mechanically and in addition coupled electrically and optically. In modifications not comprising energy consuming components like a stimulator 8 powered by the on-board energy source only the optical coupling provided here by or via the optical interface 27c could be present. In other modifications, without optical interface e.g. without any optical component but with a powered component, only the electrical coupling could be present. The mechanical interconnection however remains in both modifications.

Figure 5 is a diagrammatical illustration of an infusion system of a first embodiment comprising an infusion pump 30 which can be worn e.g. under the clothing on a belt or in a pocket, and the infusion set 1, 2 of the first example embodiment, which can be attached at the infusion site on the skin independently of the location of the infusion pump 30. The fluidic connection is established, as explained in connection with figures 1 to 4, by means of flexible tubing 3. The infusion pump 30 comprises a reservoir 32 containing the medicament, for example an ampoule, a feeding means 33 for feeding the medicament from the reservoir 32 via the tubing 3 and through the infusion set 1, 2 into the body tissue. Feeding means 33 is powered by an energy source 31 on-board the infusion pump 30. In alternative embodiments, the energy source 31 can be arranged external of the infusion pump 30, for example as an integral part of a remote control unit connected to the infusion pump 30 by wire or wireless for wired or wireless power supply or wired or wireless signal communication. A preferred type of such a combination of a light-weight infusion pump and remote control unit is disclosed in EP 1 633 414 incorporated by reference herein. Feeding means 33 is controlled by an electronic control unit 35 on-board the infusion pump 30. Parts of the control unit 35 may alternatively be external as disclosed in EP 1 633 414.

The infusion pump 30 and the infusion set 1, 2 are coupled by wireless communication 39. The communication 39 is bidirectional in the example embodiment 20. The infusion set 1, 2 comprises an on-board transceiver 28 communicating, in the coupled state, bidirectionally with a transceiver 38 on-board the infusion pump 30. In modifications the communication 39 may be unidirectional, the infusion set 1, 2 either only receiving control signals from or only transmitting sensor signals or data or other information or alarm signals to the infusion pump 30. The communication 39 can for example be a RF communication, in particular a bluetooth communication. If the infusion pump 30 can transmit control signals to the infusion set 1, 2, via either a bidirectional communication 39 or an only unidirectional one, the control signals can be signals to activate the stimulator 8, optionally to deactivate the stimulator 8, or to activate the delivery supervisory installation 7, optionally to deactivate it. If the infusion set 1, 2 is transmitting signals or data to the infusion pump 30, preferably via the bidirectional communication 39 or instead by a only unidirectional one, such signals or data can in particular be sensor signals or data of the delivery supervisory installation 7, for example to inform the user of a malfunction or of proper flow of the medicament. The signals or data may be recorded in a memory of the infusion pump 30.

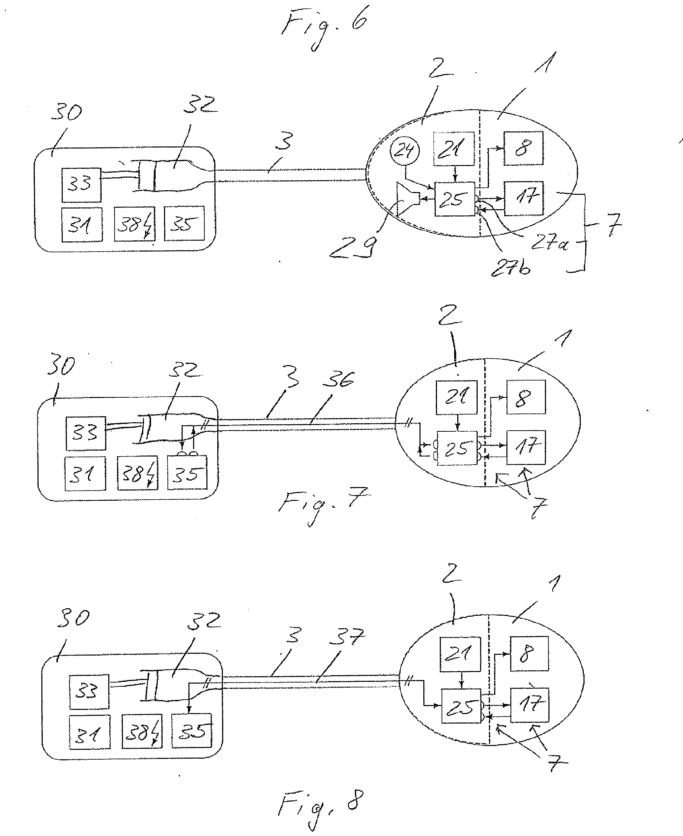

Figure 6 is a diagrammatical illustration of an infusion system according to a second embodiment of the invention. The infusion system comprises an infusion pump 30 and an infusion set 1, 2 which can be attached on the skin locally independent of the infusion pump 30, as in the first embodiment. The infusion set 1, 2 differs from that of the first embodiment in that it is not provided with the capability of signal or data transmission to or from the infusion pump 30. There is neither a transmitter nor a receiver disposed at the infusion set 1, 2 of the second embodiment. The infusion set 1, 2 is only fluidically connected to the infusion pump 30.

The infusion set 1, 2 of the second embodiment comprises a manipulator 24 for manually activating the stimulator 8 which is, except for the capability of being activated manually, identical to that of the first embodiment. The on-board manipulator 24 replaces the remote actuation by means of the infusion pump. The infusion set 1, 2 of the second embodiment further comprises an acoustic alarm means 29. The acoustic alarm means 29 could be replaced by a vibratory alarm means or by an acoustic and vibratory alarm means. The manipulator 24 and the alarm means 29 are coupled with the electronic control unit 25. The stimulator 8 is activated by actuating the manipulator 24 which can be provided e.g. as a pushbutton. Actuation of the manipulator 24 activates the stimulator 8 and, at the same time, a time lapse system, e.g. a clock, also disposed at the infusion set 1, 2, preferably as an integral part of the control unit 25. At expiration of a certain time limit, e.g. 5 to 20 seconds, predetermined by the time lapse system the alarm means 41 releases an acoustical alarm. The person perceiving the alarm signal will then actuate the infusion pump 30, directly or by means of a remote control unit if such is present, and the infusion pump 30 will deliver a bolus of medicament. The infusion pump 30 is equipped with a transmitter or receiver or preferably a transceiver 38, however, this communication means 38 does not communicate with the infusion set 1, 2. It is provided for communication with e.g. a remote control unit for remotely controlling the infusion pump 30, if applicable, or with a separate health parameter monitoring system, if the infusion system 1, 2, 30 is part of an infusion and monitoring system.

The manipulator 24 can alternatively or preferably in addition be coupled to the delivery supervisory installation 7, preferably via the control unit 25. In such embodiments an actuation of the manipulator 24 will either activate the delivery supervisory installation 7, in the example embodiment the non-contact sensor 27, or both, the stimulator 8 and the delivery supervisory installation 7.

The infusion set 1, 2 of the first embodiment can also be equipped with an on-board manipulator 24 or an on-board alarm means 29, one or both of these components being provided in addition to the remote communication capability of the infusion set 1, 2 of the first embodiment.

Figure 7 illustrates an infusion system of a third embodiment in which the infusion set 1, 2 is coupled to the infusion pump 30 via the tubing 3 not only fluidically but also optically for signal or data transmission, preferably a bidirectional signal or data transmission. The tubing 3 is provided with a communication link 36, e.g. optical fibres, connecting the infusion set 1, 2 to the infusion pump 30 via respective optical couplings at the ends of the tubing 3. In the example embodiment, the control unit 25 is optically coupled with the control unit 35 of the infusion pump 30.

Figure 8 is a diagrammatical illustration of an infusion system of a fourth embodiment in which the tubing 3 provides for the fluidic connection and also for signal or data transmission between the infusion pump 30 and the infusion set 1, 2. The only difference with respect to the third example embodiment is that in the fourth embodiment the signal or data transmission is accomplished by a galvanic communication link 37 provided as a part of the tubing 3.

The infusion sets 1, 2 of the second, third and fourth embodiments comply with the infusion set 1, 2 of the first embodiment in all respects not explicitly mentioned above.

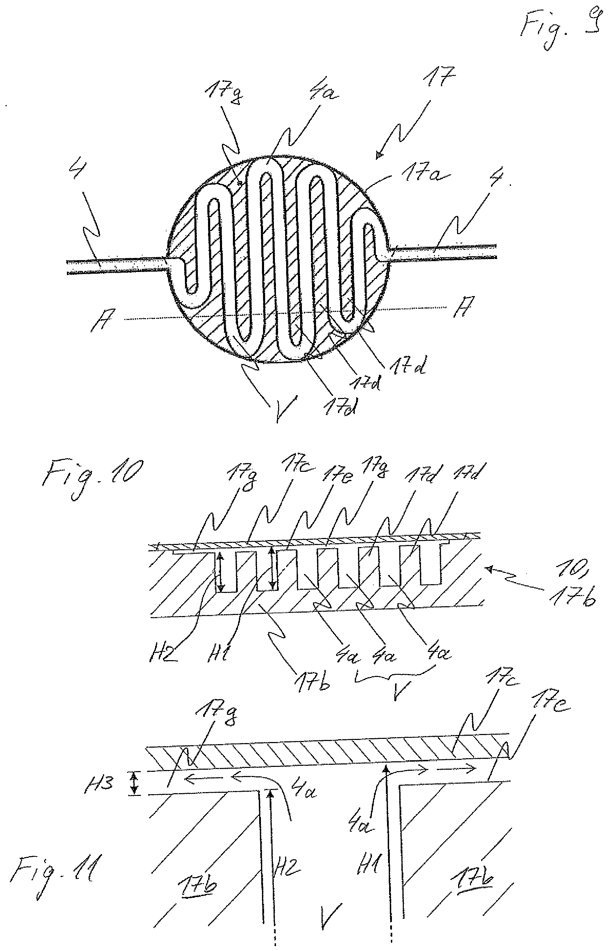

Figures 9 to 11 show an advantageous example embodiment of the contact sensor element or unit 17. Sensor means 17 comprises a micro-fluidic chamber 17a. The oval, in top view for example circular chamber 17a comprises a bottom substrate 17b formed in the base portion of the basic structure 10, 14 of the disposable part 1 and a top cover 17c. The top cover 17c is spaced from the bottom substrate 17b by a certain height HI, thus defining an inner volume V of the chamber 17a. Eight walls 17d are arranged in the fluid chamber 17a, and define a meander-like fluid channel 4a that runs from an inlet to an outlet of feeding line 4. The first connector 15 and the cannula 11 are connected by the fluid channel 4a which forms a section of the feeding line 4.

The height H2 of the walls 17d is less that the overall height H1 of the chamber 17a. As a result there is a fluid gap 17g between the top cover 17c and the upper surface 17e of the walls 17d, with a height H3 = H1 - H2. The dimensions of the chamber 17a and the walls 17d, particularly the heights HI, H2, H3 are chosen such that there are non-negligible capillary forces acting on a fluid present in the micro-fluidic chamber 17a. Fluid in the fluid channel 4a will be dragged by said capillary forces into the fluid gap 17g.

The specific dimensions depend on the one hand on the liquid used, and on the other hand on the properties the surfaces 17e of the top cover 17c and the top of the walls 17d, since this will eventually define the interface tensions between liquid, surfaces, and gas/air in the chamber 17a, which then will define the effective capillary forces for a certain geometric setting of the micro-fluidic chamber 17a. Since in most cases liquid medicaments are aqueous solutions, it is preferable that at least the most relevant surfaces, namely the top surface 17e of the wall 17d and the surface of the top cover 17c facing toward surface 17e are hydrophilic, with a contact angle < 90 °, in order to increase the overall capillary effect. For aqueous liquids a preferred range for the height H3 of the gap 17g lies between 20 and 200 µm, and preferably between 50 and 150 µm.

The dimensions of the chamber 17a and the fluid channel 4a are less critical. A typical diameter of a micro-fluidic chamber 17a may for example lie between about 2 to 10 mm. The fluid channel 4a may have a width of for example 0.1 to 1 mm, while the height H2 of the walls 17d lies in a range between 0.25 to 5 mm, and preferred lies between 0.5 and 1 mm. The aspect ratio between the width of the fluid channel 4a and the height H2 can lie between 0.25 and 5, and is preferably about 1.

When the micro-fluidic chamber 17a is filled through the inlet with a liquid, the liquid will flow essentially along the fluid channel 4a. The capillary forces will drag liquid in the fluid channel 4a into the adjacent sections of the gap 17g, effectively supplanting air present in the gap. It is energetically much more favorable for air to form spherical bubbles with minimum surface toward the hydrophilic surroundings, and thus no air bubbles remaining in the gap 17g.
The capabilities of the micro-fluidic chamber 17a are independent from its orientation in space. Since the capillary forces and interface tensions responsible for the smooth filling of the gap are much stronger than the gravitational force acting on the liquid, and the buoyancy force acting on the air bubbles in the liquid, the micro-fluidic chamber finally will be completely filled with liquid independent on its orientation. Thus the filling behavior of such a micro-fluidic chamber are predictable and reproducible.

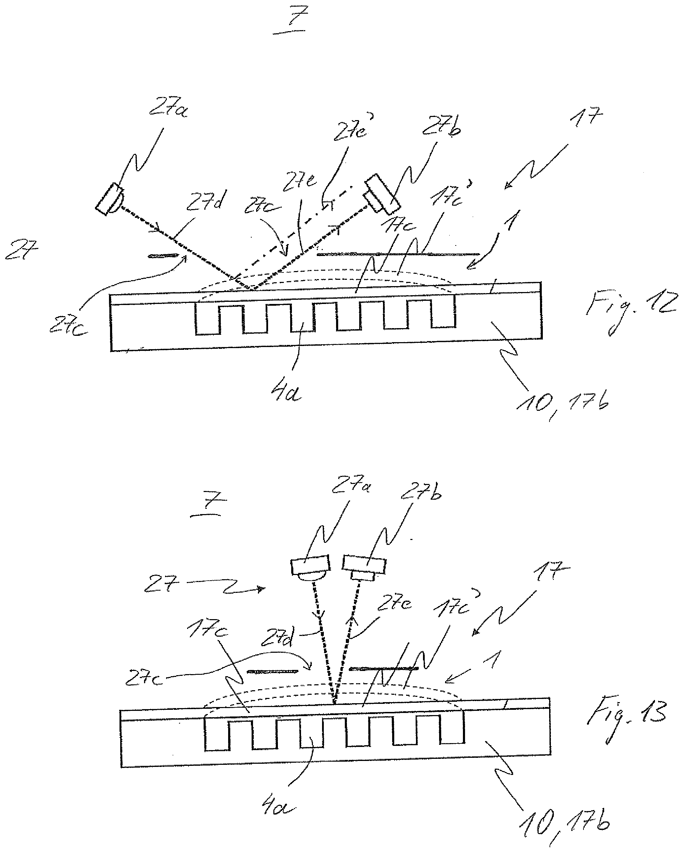

Two example embodiments of the delivery supervisory installation 7 including the contact sensor element or unit 17 of figures 9 to 11 are shown in Figures 12 and 13, both based on optical principles. The top cover 17c is a flexible, resilient membrane sealed to the base member 10 along the outer rim of the chamber 17a. The light emitter 27a, such as for example a light emitting diode (LED) or a laser diode, and the light receiver 27b, such as for example a photo diode or a photo transistor, are arranged such that an incident light beam 27d emitted by the emitter 27a is reflected by the surface of the top cover 17c toward the receiver 27b, where it is detected. The top cover 17c may be metal vapor coated to increase reflection. A metal coating for increasing reflection may also be realized by galvanic or chemical deposition or by a sandwich structure of a metal and a non-metal layer. When the top cover 17c bulges under a positive pressure difference (dashed lines 17c') the reflected light beam 27e does not impinge any longer on receiver 27b. In such an embodiment the detection system thus delivers a binary on/off signal correlated to a certain pressure threshold, which can be used by a control unit of the distant infusion pump. Such a relatively simple system is completely sufficient to detect occlusion in a fluid line. To achieve a higher resolution in the pressure values, a sensor receiver array can be used instead of a single sensor receiver 27b. This embodiment is preferable if the pressure values are used by the control unit 25 or a control unit of the pump to calculate the current flow of liquid and the administered dose of liquid medicament or for detecting a steady pressure increase over time as indication for an occluded cannula.

Another variant of the optical delivery supervisory installation 7 is shown in figure 13, where the light emitter 27a and the receiver 27b are arranged in such a way that the reflected light beam 27e will fall onto the receiver 27b independent of a displacement of the top cover membrane 17c. The position of the surface of the top cover 17c is determined by analyzing the amplitude of the reflected light, which depends on the length of the combined light path 27d, 27e.

In further developments, the non-contact sensor 27 can comprise two or more of the receivers 27b. The receivers 27b are arranged such that the reflected light beam 27e will always fall onto at least one of the receivers 27b at any state of deformation of the membrane 17c. A non-contact sensor 27 modified this way guarantees that a sensor output signal is created at any pressure level in the feeding line 4, or to be more precise in its channel 4a.

In the embodiments with an optical supervisor installation 7, e.g. the first embodiment or the embodiments according to figures 9 to 13, the disposable part 1 and the reusable part 2 are coupled optically via the respective optical interface 27c which is simply a break-through provided by respective openings at the mating surfaces of the parts 1 and 2.

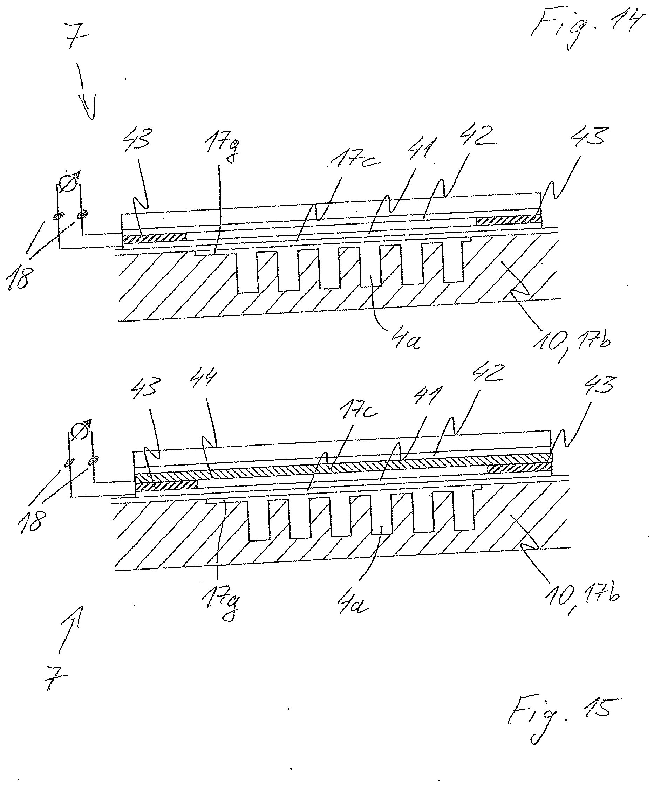

Further advantageous embodiments of a delivery supervisory installation 7 operated as a pressure sensor with a contact sensor element or unit, e. g. the micro-fluidic chamber 17a with a top cover formed as an elastic membrane 17c, are shown in figures 14 and 15. Here the displacement of the flexible membrane 17c is determined by measuring a capacitance. The delivery supervisory installation 7 of these embodiments are disposed at the disposable part 1 and electrically connected via electric connection means 18 with the reusable part 2 firstly for electrically powering the contact sensor element or unit 17 and secondly for transmitting the sensor output signals via the electric connecting means 18 to a capacitance measuring means disposed preferably at the reusable part 2. So the electric connection means 18 is the interface for the transmission of both, electric energy to the contact sensor element or unit 17 and sensor output signals to the signal processing and control unit of the infusion set, which can in particular be formed by a control unit 25 like that of the example embodiments of figures 1 to 8 but adapted to the delivery supervisory installation 7 of the capacitance type, which modified control unit includes a capacitance measuring means. In the example embodiment a first capacitor electrode 41, for example a thin metal foil, is arranged adjacent to the flexible top cover membrane 17c. Alternatively the membrane 17c itself can be realized as a capacitor electrode 41, for example by coating it with a conducting material.

In the embodiment in figure 14 insulating spacer elements 43 define a distance between said first capacitor electrode 41 and a second capacitor electrode 42, located on top of the spacer elements 43 and the first capacitor electrode 41. The capacitor electrodes 41 and 42 are electrically isolated from each other, and thus act as a capacitor with a capacitance C, which can be measured. With increasing internal pressure in the chamber 17a, the flexible, resilient membrane 17a bulges outwards. The first capacitor electrode 41 is displaced towards the second capacitor electrode 42. As a result the capacitance C increases, which can be detected and used to determine the deformation of the membrane 17c and the internal pressure in the chamber 17a causing said deformation, respectively.

When the internal pressure is high enough the first capacitor electrode 41 will eventually touch the second capacitor electrode 42, and the ohmic resistance R between the two layers drops to zero. This event can also be detected by suitable electronic means, e. g. the on-board control unit 25, and can be used - in addition or as an alternative to the capacitance - as an input for a control system of the infusion system.

Another variant of a capacitive delivery supervisory installation 7 is shown in figure 15, where an additional insulating layer 44 is arranged between the spacer elements 43 and the second capacitor electrode 42. Said additional layer 44 inhibits a short-circuit between the capacitor electrodes 41 and 42, which can be preferable, depending on the used capacitance measurement circuitry.

In yet another advantageous embodiment, the second capacitor electrode 42 can be located on the opposite side of the chamber 17a, below the bottom structure 17b, or integrated into the bottom structure 17b.

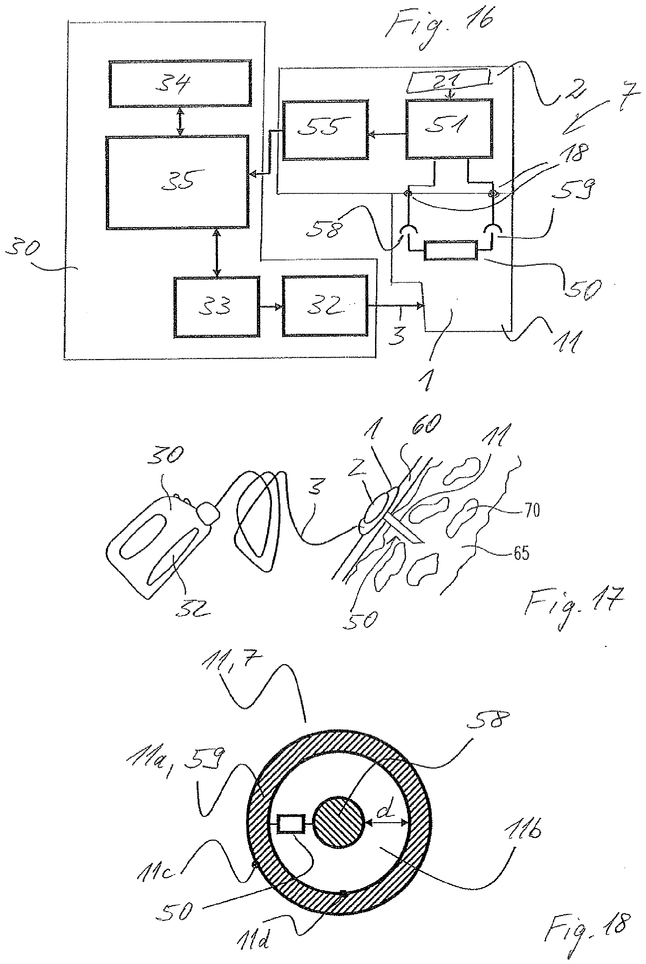

Figures 16 to 18 show a further exemplary infusion system. The infusion system comprises an infusion pump 30 with a medicament reservoir 32, feeding means 33, control unit 35 and a user interface 34, the user interface 34 being designed to indicate alarms. The infusion system furthermore comprises an infusion set with a disposable part 1 and a reusable part 2. The disposable part 1 comprises an infusion cannula 11 with the distal cannula tip. An impedance measuring means 51 and an event trigger means 55 are disposed at the reusable part 2. The infusion cannula 11 is made of medical grade stainless steel and is fluidically connected with the reservoir 32 via the infusion tubing 3. The cannula 11 further comprises two subcutaneous electrodes 58 and 59 as electrodes which are coupled via a coupling impedance 50 having an impedance value R. The coupling impedance 50 is not an electric component but is given by the medicament or subcutaneous tissue coupling the electrodes. The electrodes 58 and 59 are operatively coupled to the impedance measuring means 51. The electrodes 58 and 59 are connected with the on-board energy source 21 as disclosed for any of one of the other embodiments shown in figures 3 to 8. The electrodes 58 and 59 form the contact sensor element or unit of this delivery supervisory installation 7.

The impedance measuring means 51 is designed to measure an ohmic resistance as impedance value R and may be of any kind known in the art. The impedance measuring means 51 is operatively coupled to the event trigger means 55 which is designed to evaluate the impedance value R and generate an event trigger as described below in greater detail. Either or both of the impedance measuring means 51 or the event trigger means 55 may be, fully or partly, integral with a control unit like the control unit 25 of the embodiments of figures 1 to 8 or be disposed as additional equipment.

Upon reception of an event trigger generated by the event trigger means 55, the control unit 25 (figures 3 to 8) generates an alert or error message or a warning which is indicated to the user via the user interface 34 and preferably stops further medicament administration. The user interface 34 of the pump preferably comprises optical indicators, such as a display as well as acoustical and/or tactile indicators, such as a buzzer and/or a pager vibrator.

Most of the components of the exemplary infusion pump 30 are enclosed by a common device housing shown in figure 17, as in the other embodiments. Alternatively, the infusion pump 30 may be split into two or more separate units which are physically or operatively coupled. For example, the user interface 34 may be made by a remote controller, a cell phone, or the like, and may communicate with the control unit 35 via a wireless data interface.

The infusion cannula 11 penetrates the skin 60 in a substantially perpendicular manner and is placed in the subcutaneous tissue 65, the subcutaneous tissue 65 having interstitial fluid 70.

Figure 18 shows a cross sectional view of the infusion cannula 11, substantially being a cylindrical tube having a cannula wall 11a and an administration aperture 11b at its distal tip. The electrode 58 is a subcutaneous center electrode and is arranged in the center of the administration aperture 11b. The second electrode 59 is a subcutaneous counter electrode which is made by the cannula wall 11a. The subcutaneous center electrode 58 and the subcutaneous counter electrode 59 have a radial distance d which may be in a preferable range of e.g., 0.05mm to 0.5mm. Alternatively, the cannula 11 may be made from a non-conductive material, such as Teflon. In this, case, the subcutaneous counter electrode 59 is preferably designed as a ring or ring segment and arranged at the cannula circumferential outer or inner surface 11c or 11d.

### Reference numerals:

- 1: disposable part
- 2: reusable part
- 3: tubing
- 4: feeding line
- 4a: sensor channel
- 5: fluidic connector
- 6: locking means
- 7: delivery supervisory installation
- 8: stimulator
- 9: -
- 10: base member
- 11: cannula
- 11a: cannula wall
- 11b: cannula aperture
- 11c: outer cannula surface
- 11d: inner cannula surface
- 12a: upright structure
- 12b: upright structure
- 13: recess
- 14: base member
- 15: fluidic or first connector
- 16: mechanical or second connector
- 17: contact sensor element or unit
- 17a: chamber
- 17b: bottom substrate
- 17c: top cover, membrane
- 17d: wall
- 17e: surface
- 17g: gap
- 18: electric connection means
- 19: air degasser
- 20: cap
- 21: energy source
- 22: base member
- 23: guiding
- 24: manipulator
- 25: control unit
- 26: mechanical or third connector
- 27: non-contact sensor
- 27a: sensor emitter
- 27b: sensor receiver
- 27c: optical interface
- 27d: incident light beam
- 27e: reflected light beam
- 28: transceiver
- 29: alarm means
- 30: infusion pump
- 31: energy source
- 32: reservoir
- 33: feeding means
- 34: user interface
- 35: control unit
- 36: optical communication link
- 37: galvanic communication link
- 38: transceiver
- 39: wireless communication
- 40: -
- 41: first electrode
- 42: second electrode
- 43: spacer element
- 44: additional layer
- 50: impedance
- 51: impedance measuring means
- 55: trigger means
- 58: electrode
- 59: electrode
- 60: skin
- 65: subcutaneous tissue
- 70: interstitial fluid

- H: Height
- V: Volume

## Claims

1. Infusion set for administering a medicament delivered by an infusion pump (30) which can be carried separately from the infusion set,
a) the infusion set comprising a disposable part (1), a reusable part (2), and an electrically powered functional component (7, 8),
b) the disposable part (1) comprising:
b1) an adhesive underside for attachment at an infusion site on a person's skin,
b2) a single lumen infusion cannula (11) which projects from the underside and is the only skin piercing or penetrating element of the infusion set,
b3) a first connector (15) for fluidically connecting the cannula to the infusion pump,
b4) and a second connector (16),
c) and the reusable part (2) comprising:
c1) an energy source (21) for electrically powering the functional component (7, 8),
c2) and a third connector (26) mating with the second connector (16) of the disposable part (1) to interconnect the disposable part (1) and the reusable part (2),
d) wherein the reusable part (2) is in the interconnected state fluidically isolated from the disposable part (1);
e) wherein the disposable part (1) comprises a feeding line (4) fluidically connecting an upstream end of the first connector (15) with a downstream end of the cannula (11) to feed and deliver the medicament via the disposable part (1) and bypass the reusable part (2); **characterized in that**
f) the disposable part (1) comprises a primary base member (10) and a secondary base member (14) which are formed separately and jointly fixed one to the other such as to jointly form a chamber suitable for disposing an air degasser (19) in the medicament flow; and
g) wherein:
g1) the primary base member (10) comprises the single lumen infusion cannula (11) and the second connector (16); and
g2) the secondary base member (14) comprises the first connector (15).

2. Infusion set according to the preceding claim, wherein the functional component (8) is either a stimulator (8) for heating or mechanically stimulating the tissue at the infusion site or a delivery supervisory installation (7) for supervising the delivery of the medicament by sensing a variable which is characteristic for the medicament flow downstream of or at the first connector (15).

3. Infusion set according to any of the preceding claims, wherein the functional component (8) is a stimulator (8) which forms part of the underside of the disposable part (1), preferably at least partially surrounding the cannula (11).

4. Infusion set according to any of the preceding claims, wherein the functional component (7) is a delivery supervisory installation (7) for supervising the delivery of the medicament by sensing a variable characteristic for the medicament flow downstream of or at the first connector (15), and the delivery supervisory installation (7) comprises a contact sensor element or unit (17; 17c; 58, 59) disposed at the disposable part (1) in contact with a feeding line (4) for the medicament or directly with the medicament, the feeding line (4) fluidically connecting an upstream end of the first connector (15) with a downstream end of the cannula (11), and the contact sensor element or unit (17; 17c; 58, 59) preferably being or comprising a deformation structure (17; 17c) which deforms in dependence of a fluid pressure within the feeding line (4).

5. Infusion set according to the preceding claim, wherein the functional component (7) is a delivery supervisory installation (7) for supervising the delivery of the medicament by sensing a variable characteristic for the medicament flow downstream of or at the first connector (15), the delivery supervisory installation (7) comprising a non-contact sensor (27) for a contactless supervision of medicament flow through a feeding line (4) of the infusion set (1, 2), the feeding line (4) fluidically connecting an upstream end of the first connector (15) with a downstream end of the cannula (11), and the non-contact sensor (27) preferably being disposed at the reusable part (2).

6. Infusion set according to the combination of claims 4 and 5, wherein the contact sensor element or unit (12; 17c) is disposed at the disposable part (1) and the non-contact sensor (27) at the reusable part (2), and the non-contact sensor (27) is arranged for sensing the contact sensor element or unit (17; 17c) optically.

7. Infusion set according to any one of the preceding claims, comprising a manipulator (24) for manual manipulation of the functional component (8), preferably for activating the functional component (8), the manipulator (24) being disposed preferably at the reusable part (2).

8. Infusion set according to any one of the preceding claims and at least one of the following features:
(i) the functional component is a lifetimer for alerting the patient at expiration of a predetermined time of use of the disposable part (1), the lifetimer being preferably disposed at the reusable part (2);
(ii) the functional component is an alarm means (29) for releasing an acoustic or vibratory signal, the alarm means (29) being preferably disposed at the reusable part (2).

9. Infusion set according to any one of the preceding claims and at least one of the following features:
(i) the functional component is a motion sensor for sensing motional activity of the person, the motion sensor being preferably disposed at the reusable part (2);
(ii) the functional component is a perspiration sensor for sensing perspiration at a surface of the skin, which is in contact with the underside of the disposable part (1);
(iii) the functional component is a temperature sensor for sensing an ambient temperature or skin temperature, a temperature sensor for sensing skin temperature being disposed preferably at the underside of the disposable part (1);
(iv) the functional component is a stimulator (8) for heating the tissue at the infusion site, and the infusion set comprises a temperature sensor for supervising the stimulator (8);
(v) the functional component is a skin contact sensor for sensing whether the infusion set is in contact with the skin or not.

10. Infusion set according to any one of the preceding claims, wherein the reusable part (2) comprises a transmitter or receiver (23) for transmitting signals to or receiving signals from the infusion pump (30), preferably for wireless transmission, the energy source (21) electrically powers the transmitter or receiver (28) and the transmitter or receiver (28) is coupled with the functional component (7, 8) for infusion-set-internal signal communication.

11. Infusion set according to any one of the preceding claims, wherein the reusable part (2) comprises a signal processing or control unit (25) for controlling the functional component (7, 8) or processing signals received from the functional component (7), and the energy source (21) electrically powers the signal processing or control unit (25), the functional component (7, 8) preferably being powered via the signal processing or control unit (25).

12. Infusion set according to any one of the preceding claims, and at least one of the following features:
(i) the infusion set (1, 2) is electrically or optically coupable or coupled with the infusion pump (30) for signal or data transmission via fluidic tubing (3);
(ii) the infusion set (1, 2) comprises flexible tubing for connecting the infusion set (1, 2) fluidically with the infusion pump (30), preferably for connecting it releasably with the infusion pump (38).

13. Infusion set according to any one of the preceding claims and at least one of the following features:
(i) the reusable part (2) is interconnected mechanically with the disposable part (1), and is in addition to the mechanical interconnection coupable or coupled either only electrically or only optically or only electrically and optically with the disposable part (1);
(ii) the reusable part (2) is interconnected with the disposable part (1) only mechanically.

14. Infusion set according to any one of the preceding claims, wherein the disposable part (1) comprises a base (10, 14), preferably a flat base, which forms the underside and an upper side opposite to the underside, the first connector (15) extending at least essentially parallel to the upper side, and the reusable part (2) is attached to the upper side of the disposable part (1), the first connector (15) preferably protruding from an upright structure (12a, 12b) which protrudes from the upper side of the base (10, 14).

15. Infusion system, comprising:
a) an infusion set (1, 2) according to any one of the preceding claims,
b) an infusion pump (30) with a medicament reservoir (32), feeding means (33) for feeding medicament from the reservoir, and an energy source (31) for powering the feeding means,
c) and flexible tubing (3) connecting the infusion pump (30) with the infusion set (1, 2) to administer the medicament from the reservoir (32) via the cannula (1 I) of the infusion set (1, 2), the tubing (3) being directly connected releasably or unreleasably with the disposable part (2) and preferably being connected releasably with the infusion pump (30),
d) optionally, a monitoring system including a sensor, preferably a glucose sensor, for sensing a therapy relevant health parameter of the person, preferably in-vivo, the sensor being separate from the infusion set (1, 2).

## Patentansprüche

1. Infusionsset zur Verabreichung eines Arzneimittels, das von einer Infusionspumpe (30) abgegeben wird, die vom Infusionsset getrennt getragen werden kann,
a) wobei das Infusionsset ein Einwegteil (1), ein Mehrwegteil (2) und eine elektrisch betriebene Komponente (7, 8) umfasst,
b) wobei das Wegwerfteil (1) Folgendes umfasst:
b1) eine klebende Unterseite zur Befestigung an einer Infusionsstelle auf der Haut einer Person,
b2) eine einlumige Infusionskanüle (11), die aus der Unterseite vorsteht und das einzige Haut durchstechende oder durchdringende Element des Infusionssets ist,
b3) einen ersten Konnektor (15), um die Kanüle strömungstechnisch an die Infusionspumpe anzuschließen,
b4) und einen zweiten Konnektor (16),
c) und ein Mehrwegteil (2) Folgendes umfasst:
c1) eine Energiequelle (21), um die Funktionskomponente (7, 8) elektrisch zu betreiben,
c2) und einen dritten Konnektor (26), der zum zweiten Konnektor (16) des Einwegteils (1) passt, um das Einwegteil (1) und das Mehrwegteil (2) miteinander zu verbinden,
d) wobei das Mehrwegteil (2) im verbundenen Zustand strömungstechnisch vom Einwegteil (1) isoliert ist;
e) wobei das Einwegteil (1) eine Zuführleitung (4) umfasst, die strömungstechnisch ein stromaufwärtiges Ende des ersten Konnektors (15) mit einem stromabwärtigen Ende der Kanüle (11) verbindet, um das Arzneimittel über das Einwegteil
(1) zuzuführen und abzugeben und das Mehrwegteil
(2) zu umgehen;
**dadurch gekennzeichnet, dass**
f) das Einwegteil (1) ein primäres Basiselement (10) und ein sekundäres Basiselement (14) umfasst, die getrennt ausgebildet und aneinander befestigt sind, sodass sie zusammen eine Kammer bilden, die geeignet ist, um einen Luftentgaser (19) im Arzneimittelfluss anzuordnen; und
g) wobei:
g1) das primäre Basiselement (10) die einlumige Infusionskanüle (11) und den zweiten Konnektor (16) umfasst; und
g2) das sekundäre Basiselement (14) den ersten Konnektor (15) umfasst.

2. Infusionsset nach dem vorhergehenden Anspruch, wobei die Funktionskomponente (8) entweder ein Stimulator (8) ist, um das Gewebe an der Infusionsstelle zu erwärmen oder mechanisch zu stimulieren, oder eine Abgabeüberwachungsvorrichtung (7) zum Überwachen der Abgabe des Arzneimittels durch Abtasten einer Variablen, die für den Arzneimittelfluss stromabwärts vom oder am ersten Konnektor (15) charakteristisch ist.

3. Infusionsset nach einem der vorstehenden Ansprüche, wobei die Funktionskomponente (8) ein Stimulator (8) ist, der Teil der Unterseite des Einwegteils (1) ist, vorzugsweise mindestens teilweise die Kanüle (11) umgebend.

4. Infusionsset nach einem der vorstehenden Ansprüche, wobei die Funktionskomponente (7) eine Abgabeüberwachungsvorrichtung (7) zum Überwachen der Abgabe des Arzneimittels durch Abtasten einer Variablen ist, die für den Arzneimittelfluss stromabwärts vom oder am ersten Konnektor (15) charakteristisch ist, und die Abgabeüberwachungsvorrichtung (7) ein Kontaktsensorelement oder eine Kontaktsensoreinheit (17; 17c; 58, 59) umfasst, die am Einwegteil (1) in Kontakt mit einer Zuführleitung (4) für das Arzneimittel oder direkt in Kontakt mit dem Arzneimittel angeordnet ist, wobei die Zuführleitung (4) strömungstechnisch ein stromaufwärtiges Ende des ersten Konnektors (15) mit einem stromabwärtigen Ende der Kanüle (11) verbindet, und wobei das Kontaktsensorelement oder die Kontaktsensoreinheit (17; 17c; 58, 59) vorzugsweise eine Verformungsstruktur (17; 17c) ist oder umfasst, die je nach Fluiddruck innerhalb der Zuführleitung (4) verformt.

5. Infusionsset nach dem vorhergehenden Anspruch, wobei die Funktionskomponente (7) eine Abgabeüberwachungsvorrichtung (7) zur Überwachung der Abgabe des Arzneimittels durch Abtasten einer Variablen ist, die für den Arzneimittelfluss stromabwärts zum oder am ersten Konnektor (15) charakteristisch ist, wobei die Abgabeüberwachungsvorrichtung (7) einen Nicht-Kontakt-Sensor (27) für eine kontaktlose Überwachung des Arzneimittelflusses durch eine Zuführleitung (4) des Infusionssets (1, 2) umfasst, wobei die Zuführleitung (4) strömungstechnisch ein stromaufwärtiges Ende des ersten Konnektors (15) mit einem stromabwärtigen Ende der Kanüle (11) verbindet, und der Nicht-Kontakt-Sensor (27) vorzugsweise am Mehrwegteil (2) angeordnet ist.

6. Infusionsset nach Kombination der Ansprüche 4 und 5, wobei das Kontaktsensorelement oder die Kontaktsensoreinheit (12; 17c) am Einwegteil (1) und der Nicht-Kontakt-Sensor (27) am Mehrwegteil (2) angeordnet ist, und der Nicht-Kontakt-Sensor (27) angeordnet ist, um das Kontaktsensorelement oder die Kontaktsensoreinheit (17; 17c) optisch abzutasten.

7. Infusionsset nach einem der vorstehenden Ansprüche, umfassend einen Manipulator (24) zur manuellen Manipulation der Funktionskomponente (8), vorzugsweise zur Aktivierung der Funktionskomponente (8), wobei der Manipulator (24) vorzugsweise am Mehrwegteil (2) angeordnet ist.

8. Infusionsset nach einem der vorstehenden Ansprüche und mindestens eins der folgenden Merkmale:
(i) Die Funktionskomponente ist ein Lifetimer, um den Patienten bei Ablauf einer vorgegebenen Anwendungszeit des Einwegteils (1) zu warnen, wobei der Lifetimer vorzugsweise am Mehrwegteil (2) angeordnet ist;
(ii) Die Funktionskomponente ist eine Warnvorrichtung (29), um ein akustisches oder vibratorisches Signal auszulösen, wobei die Warnvorrichtung (29) vorzugsweise am Mehrwegteil (2) angeordnet ist.

9. Infusionsset nach einem der vorstehenden Ansprüche und mindestens eins der folgenden Merkmale:
(i) Die Funktionskomponente ist ein Bewegungssensor zum Abtasten von Bewegungsaktivität der Person, wobei der Bewegungssensor vorzugsweise am Mehrwegteil (2) angeordnet ist;
(ii) die Funktionskomponente ist ein Schweißsensor zum Abtasten von Schweiß auf einer Fläche der Haut, die in Kontakt mit der Unterseite des Einwegteils (1) steht;
(iii) die Funktionskomponente ist ein Temperatursensor zum Abtasten einer Umgebungstemperatur oder Hauttemperatur, wobei ein Temperatursensor zum Abtasten von Hauttemperatur vorzugsweise auf der Unterseite des Einwegteils (1) angeordnet ist;
(iv) die Funktionskomponente ist ein Stimulator (8) zum Erwärmen des Gewebes an der Infusionsstelle, und das Infusionsset umfasst einen Temperatursensor zum Überwachen des Stimulators (8) ;
(v) die Funktionskomponente ist ein Hautkontaktsensor, um abzutasten, ob das Infusionsset in Kontakt mit der Haut steht oder nicht.

10. Infusionsset nach einem der vorstehenden Ansprüche, wobei das Mehrwegteil (2) einen Sender oder Empfänger (23) zum Übertragen von Signalen an oder Empfangen von Signalen aus einer Infusionspumpe (30), vorzugsweise zur drahtlosen Übertragung, umfasst, wobei die Energiequelle (21) den Sender oder Empfänger (28) elektrisch betreibt, und der Sender oder Empfänger (28) mit der Funktionskomponente (7, 8) zur internen Infusionssetsignalkommunikation verbunden ist.

11. Infusionsset nach einem der vorstehenden Ansprüche, wobei das Mehrwegteil (2) eine Signalverarbeitungs- oder Steuereinheit (25) umfasst, um die Funktionskomponente (7, 8) zu steuern oder von der Funktionskomponente (7) empfangene Signale zu verarbeiten, und die Energiequelle (21) die Signalverarbeitungs- oder Steuereinheit (25) elektrisch betreibt, wobei die Funktionskomponente (7, 8) vorzugsweise über die Signalverarbeitungs- oder Steuereinheit (25) betrieben wird.

12. Infusionsset nach einem der vorstehenden Ansprüche und mindestens eins der folgenden Merkmale:
(i) Das Infusionsset (1, 2) ist elektrisch oder optisch verbindbar oder wird mit der Infusionspumpe (30) zur Signal- oder Datenübertragung über fluidische Schlauchleitung (3) verbunden;
(ii) Das Infusionsset (1, 2) umfasst flexible Schlauchleitung, um das Infusionsset (1, 2) strömungstechnisch mit der Infusionspumpe (30) zu verbinden, vorzugsweise um es lösbar mit der Infusionspumpe (38) zu verbinden.

13. Infusionsset nach einem der vorstehenden Ansprüche und mindestens eins der folgenden Merkmale:
(i) Das Mehrwegteil (2) ist mechanisch mit dem Einwegteil (1) verbunden und ist zusätzlich zur mechanischen Verbindung mit dem Einwegteil (1) verbindbar oder entweder nur elektrisch oder nur optisch oder nur elektrisch und optisch verbunden;
(ii) Das Mehrwegteil (2) ist mit dem Einwegteil (1) nur mechanisch verbunden.

14. Infusionsset nach einem der vorstehenden Ansprüche, wobei das Einwegteil (1) eine Basis (10, 14), vorzugsweise eine flache Basis, umfasst, welche die Unterseite und eine Oberseite gegenüber der Unterseite bildet, wobei der erste Konnektor (15) sich mindestens im Wesentlichen parallel zur Oberseite erstreckt und das Mehrwegteil (2) an der Oberseite des Einwegteils (1) befestigt ist, der erste Konnektor (15) vorzugsweise aus einer aufrechten Struktur (12a, 12b) vorsteht, die aus der Oberseite der Basis (10, 14) vorsteht.

15. Infusionssystem umfassend:
(a) Ein Infusionsset (1, 2) nach einem der vorstehenden Ansprüche,
(b) eine Infusionspumpe (30) mit einem Arzneimittelbehälter (32), Zuführmittel (33), um Arzneimittel aus dem Behälter zuzuführen, und eine Energiequelle (31), um das Zuführmittel zu betreiben,
(c) und flexible Schlauchleitung (3), welche die Infusionspumpe (30) mit dem Infusionsset (1, 2) verbindet, um das Arzneimittel aus dem Behälter (32) über die Kanüle (11) des Infusionssets (1, 2) zu verabreichen, wobei die Schlauchleitung (3) direkt lösbar oder nicht lösbar mit dem Einwegteil (2) verbunden ist und vorzugsweise lösbar mit der Infusionspumpe (30) verbunden ist,
(d) wahlweise ein Überwachungssystem einschließlich eines Sensors, vorzugsweise ein Glukosesensor, zum Erkennen eines therapierelevanten Gesundheitsparameters der Person, vorzugsweise in vivo, wobei der Sensor vom Infusionsset (1, 2) getrennt ist.

## Revendications

1. Trousse de perfusion pour administrer un médicament délivré par une pompe à perfusion (30) qui peut être portée séparément de la trousse de perfusion,
a) la trousse de perfusion comprenant une partie jetable (1), une partie réutilisable (2) et une composante fonctionnelle alimentée électriquement (7, 8),
b) la partie jetable (1) comprenant :
b1) une face de dessous adhésive pour la fixation sur un site de perfusion sur la peau d'une personne,
b2) une canule de perfusion à lumière simple (11) qui fait saillie de la face de dessous et est le seul élément de la trousse de perfusion perçant ou pénétrant la peau,
b3) un premier connecteur (15) pour connecter fluidiquement la canule à la pompe de perfusion,
b4) et un deuxième connecteur (16),
c) et la partie réutilisable (2) comprenant :
c1) une source d'énergie (21) pour alimenter la composante fonctionnelle (7, 8) électriquement,
c2) et un troisième connecteur (26) s'accouplant au deuxième connecteur (16) de la partie jetable (1) pour interconnecter la partie jetable (1) et la partie réutilisable (2),
d) dans lequel la partie réutilisable (2) est, dans l'état interconnecté, isolée fluidiquement de la partie jetable (1) ;
e) dans lequel la partie jetable (1) comprend une ligne d'alimentation (4) connectant fluidiquement une extrémité en amont du premier connecteur (15) à une extrémité en aval de la canule (11) pour alimenter et délivrer le médicament par le biais de la partie jetable (1) et contourner la partie réutilisable (2) ;
**caractérisé en ce que**
f) la partie jetable (1) comprend un membre de base principal (10) et un membre de base secondaire (14) qui sont formés séparément et fixés de manière conjointe l'un à l'autre de façon à former conjointement une chambre appropriée pour disposer un dégazeur d'air (19) dans le flux de médicament ; et
g) dans lequel :
g1) le membre de base principal (10) comprend la canule de perfusion à lumière simple (11) et le deuxième connecteur (16) ; et
g2) le membre de base secondaire (14) comprend le premier connecteur (15).

2. Trousse de perfusion selon la revendication précédente, dans lequel la composante fonctionnelle (8) est soit un stimulateur (8) pour chauffer ou stimuler mécaniquement le tissu sur le site de perfusion soit une installation de surveillance de délivrance (7) pour surveiller la délivrance du médicament en détectant une variable qui est caractéristique du flux de médicament en aval du ou sur le premier connecteur (15).

3. Trousse de perfusion selon l'une quelconque des revendications précédentes, dans lequel la composante fonctionnelle (8) est un stimulateur (8) qui fait partie de la face de dessous de la partie jetable (1), entourant au moins partiellement la canule (11) de préférence.

4. Trousse de perfusion selon l'une quelconque des revendications précédentes, dans lequel la composante fonctionnelle (7) est une installation de surveillance de délivrance (7) pour surveiller la délivrance du médicament en détectant une variable qui est caractéristique du flux de médicament en aval du ou sur le premier connecteur (15), et l'installation de surveillance de délivrance (7) comprend un élément ou une unité de capteur de contact (17 ; 17c ; 58, 59) disposé(e) sur la partie jetable (1) en contact avec une ligne d'alimentation (4) pour le médicament ou directement avec le médicament, la ligne d'alimentation (4) reliant fluidiquement une extrémité en amont du premier connecteur (15) à une extrémité en aval de la canule (11), et l'élément ou l'unité de capteur de contact (17; 17c ; 58, 59) étant ou comprenant de préférence une structure à déformation (17 ; 17c) qui se déforme en fonction d'une pression du fluide à l'intérieur de la ligne d'alimentation (4) .

5. Trousse de perfusion selon la revendication précédente, dans lequel la composante fonctionnelle (7) est une installation de surveillance de délivrance (7) pour surveiller la délivrance du médicament en détectant une variable qui est caractéristique du flux de médicament en aval du ou sur le premier connecteur (15), l'installation de surveillance de délivrance (7) comprenant un capteur de non-contact (27) pour une surveillance sans contact du flux de médicament à travers une ligne d'alimentation (4) de la trousse de perfusion (1, 2), la ligne d'alimentation (4) connectant fluidiquement une extrémité en amont du premier connecteur (15) à une extrémité en aval de la canule (11), et le capteur de non-contact (27) étant disposé de préférence sur la partie réutilisable (2).

6. Trousse de perfusion selon la combinaison des revendications 4 et 5, dans lequel l'élément ou l'unité de capteur de contact (12 ; 17c) est disposé(e) sur la partie jetable (1) et le capteur de non-contact (27) sur la partie réutilisable (2), et le capteur de non-contact (27) est agencé pour détecter l'élément ou l'unité de capteur de contact (17 ; 17c) de manière visuelle.

7. Trousse de perfusion selon l'une quelconque des revendications précédentes, comprenant un manipulateur (24) pour la manipulation manuelle de la composante fonctionnelle (8), de préférence pour activer la composante fonctionnelle (8), le manipulateur (24) étant disposé de préférence sur la partie jetable (2).

8. Trousse de perfusion selon l'une quelconque des revendications précédentes et au moins une des caractéristiques suivantes :
(i) la composante fonctionnelle est une horloge pour alerter le patient à l'expiration d'un temps prédéterminé d'utilisation de la partie jetable (1), l'horloge étant de préférence disposée sur la partie réutilisable (2) ;
(ii) la composante fonctionnelle est un moyen d'alarme (29) pour émettre un signal acoustique ou vibratoire, le moyen d'alarme (29) étant de préférence disposé sur la partie réutilisable (2) .

9. Trousse de perfusion selon l'une quelconque des revendications précédentes et au moins une des caractéristiques suivantes :
(i) la composante fonctionnelle est un capteur de mouvement pour détecter l'activité de mouvement de la personne, le capteur de mouvement étant de préférence disposé sur la partie réutilisable (2) ;
(ii) la composante fonctionnelle est un capteur de transpiration pour détecter la transpiration à une surface de la peau qui est en contact avec la face de dessous de la partie jetable (1) ;
(iii) la composante fonctionnelle est un capteur de température pour détecter une température ambiante ou une température de la peau, un capteur de température pour détecter la température de la peau étant disposé de préférence sur la face de dessous de la partie jetable (1) ;
(iv) la composante fonctionnelle est un stimulateur (8) pour chauffer le tissu sur le site de perfusion, et la trousse de perfusion comprend un capteur de température pour surveiller le stimulateur (8) ;
(v) la composante fonctionnelle est un capteur de contact cutané pour détecter si la trousse de perfusion est en contact avec la peau ou non.

10. Trousse de perfusion selon l'une quelconque des revendications précédentes, dans lequel la partie réutilisable (2) comprend un émetteur ou un récepteur (23) pour émettre des signaux vers, ou recevoir des signaux de, la pompe à perfusion (30), de préférence pour la transmission sans fil, la source d'énergie (21) alimente électriquement l'émetteur ou le récepteur (28) et l'émetteur ou le récepteur (28) est couplé à la composante fonctionnelle (7, 8) pour la communication trousse de perfusion-signal interne.

11. Trousse de perfusion selon l'une quelconque des revendications précédentes, dans lequel la partie réutilisable (2) comprend une unité de traitement ou de commande du signal (25) pour commander la composante fonctionnelle (7, 8) ou traiter des signaux reçus de la composante fonctionnelle (7), et la source d'énergie (21) alimente électriquement l'unité de traitement ou de commande du signal (25), la partie fonctionnelle (7, 8) étant de préférence alimentée par l'unité de traitement ou de commande du signal (25).

12. Trousse de perfusion selon l'une quelconque des revendications précédentes et au moins une des caractéristiques suivantes :
(i) la trousse de perfusion (1, 2) peut être couplé ou est couplé électriquement ou visuellement à la pompe à perfusion (30) pour la transmission de signal ou de données par tubage fluidique (3) ;
(ii) la trousse de perfusion (1, 2) comprend un tubage flexible pour connecter la trousse de perfusion (1, 2) fluidiquement à la pompe à perfusion (30), de préférence pour le connecter de manière séparable à la pompe à perfusion (38).

13. Trousse de perfusion selon l'une quelconque des revendications précédentes et au moins une des caractéristiques suivantes :
(i) la partie réutilisable (2) est interconnectée mécaniquement avec la partie jetable (1) et en plus de l'interconnexion mécanique, peut être couplée ou est couplée soit uniquement électriquement soit uniquement visuellement ou uniquement électriquement et visuellement avec la partie jetable (1) ;
(ii) la partie réutilisable (2) est interconnectée avec la partie jetable (1) uniquement mécaniquement.

14. Trousse de perfusion selon l'une quelconque des revendications précédentes, dans lequel la partie jetable (1) comprend une base (10, 14), de préférence une base plate qui forme la face de dessous et une face de dessus opposée à la face de dessous, le premier connecteur (15) s'étendant au moins essentiellement parallèle à la face de dessus, et la partie réutilisable (2) est fixée à la face de dessus de la partie jetable (1), le premier connecteur (15) faisant saillie de préférence d'une structure verticale (12a, 12b) qui fait saillie de la face de dessus de la base (10, 14).

15. Trousse de perfusion, comprenant :
a) une trousse de perfusion (1, 2) selon l'une quelconque des revendications précédentes,
b) une pompe à perfusion (30) avec un réservoir de médicament (32), un moyen d'alimentation (33) pour alimenter un médicament à partir du réservoir, et une source d'énergie (31) pour alimenter le moyen d'alimentation,
c) et un tubage flexible (3) connectant la pompe à perfusion (30) à la trousse de perfusion (1, 2) pour administrer le médicament à partir du réservoir (32) par le biais de la canule (11) de la trousse de perfusion (1, 2), le tubage (3) étant directement relié de façon libérable ou non libérable à la partie jetable (2) et étant relié de préférence de manière libérable avec la pompe à perfusion (30),
d) en option, un système de surveillance incluant un capteur, de préférence un capteur de glucose, pour détecter un paramètre de santé de la personne pertinent pour le traitement, de préférence in-vivo, le capteur étant séparé de la trousse de perfusion (1, 2).
